(19) Europäisches Patentamt
European Patent Office
Office européen des brevets

(11) **EP 4 069 181 B1**

(12) **FASCICULE DE BREVET EUROPEEN**

(45) Date de publication et mention
de la délivrance du brevet:
**06.11.2024   Bulletin 2024/45**

(21) Numéro de dépôt: **20812390.1**

(22) Date de dépôt: **02.12.2020**

(51) Classification Internationale des Brevets (IPC):
**A61K 8/02** *(2006.01)*     **A61Q 5/02** *(2006.01)*
**A61Q 19/10** *(2006.01)*     **A61K 8/9794** *(2017.01)*
**A61K 8/73** *(2006.01)*     **A61K 8/34** *(2006.01)*
**A61K 8/92** *(2006.01)*

(52) Classification Coopérative des Brevets (CPC):
**A61K 8/022; A61K 8/73; A61Q 5/02; A61Q 19/10**

(86) Numéro de dépôt international:
**PCT/EP2020/084318**

(87) Numéro de publication internationale:
**WO 2021/110768 (10.06.2021 Gazette 2021/23)**

(54) **COMPOSITIONS COSMETIQUES NETTOYANTES SOUS FORME DE POUDRE**

KOSMETISCHE REINIGUNGSMITTEL IN PULVERFORM

COSMETIC CLEANING COMPOSITIONS IN POWDER FORM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:   **04.12.2019   FR 1913768**
**09.06.2020   FR 2006002**

(43) Date de publication de la demande:
**12.10.2022   Bulletin 2022/41**

(73) Titulaire: **Yodi SAS**
**75116 Paris (FR)**

(72) Inventeur: **AZANCOT, Hélène**
**92200 NEUILLY SUR SEINE (FR)**

(74) Mandataire: **Grosset-Fournier, Chantal Catherine et al**
**Grosset-Fournier & Demachy**
**54, rue Saint-Lazare**
**75009 Paris (FR)**

(56) Documents cités:
**WO-A1-2019/023332     WO-A1-2019/092060**
**GB-A- 2 498 543     KR-B1- 101 308 959**

- **DATABASE GNPD [online] MINTEL; 18 March 2019 (2019-03-18), ANONYMOUS: "Facial Washing Powder", XP055775941, retrieved from https://www.gnpd.com/sinatra/recordpage/6408 197/ Database accession no. 6408197**
- **DATABASE GNPD [online] MINTEL; 9 February 2021 (2021-02-09), ANONYMOUS: "Purifying Charcoal Powder Cleanser with Prebiotic", XP055775931, retrieved from https://www.gnpd.com/sinatra/recordpage/8472 055/ Database accession no. 8472055**
- **DATABASE GNPD [online] MINTEL; 1 April 2020 (2020-04-01), ANONYMOUS: "Macadamia Orange Shampoo Powder", XP055776108, retrieved from https://www.gnpd.com/sinatra/recordpage/7504 051/ Database accession no. 7504051**
- **DATABASE GNPD [online] MINTEL; 18 July 2017 (2017-07-18), ANONYMOUS: "Clay-Mud Cleansing Powder", XP055775879, retrieved from https://www.gnpd.com/sinatra/recordpage/4939 273/ Database accession no. 4939273**
- **DATABASE GNPD [online] MINTEL; 2 September 2016 (2016-09-02), ANONYMOUS: "Amazonian Ritual Powder Shampoo", XP055706987, retrieved from https://www.gnpd.com/sinatra/recordpage/4251 593/ Database accession no. 4251593**

EP 4 069 181 B1

- DATABASE GNPD [online] MINTEL; 15 April 2020 (2020-04-15), ANONYMOUS: "Powder Wash", XP055776065, retrieved from https://www.gnpd.com/sinatra/recordpage/7538 435/ Database accession no. 7538435
- DATABASE GNPD [online] MINTEL; 4 June 2019 (2019-06-04), ANONYMOUS: "Deep Clear Washing Powder", XP055775906, retrieved from https://www.gnpd.com/sinatra/recordpage/6608 263/ Database accession no. 6608263
- DATABASE GNPD [online] MINTEL; 4 April 2020 (2020-04-04), ANONYMOUS: "Shower Powder with White Oyster Shell", XP055775892, retrieved from https://www.gnpd.com/sinatra/recordpage/7434 751/ Database accession no. 7434751
- DATABASE GNPD [online] MINTEL; 14 August 2019 (2019-08-14), ANONYMOUS: "Clear Wash Powder", XP055775909, retrieved from https://www.gnpd.com/sinatra/recordpage/6796 831/ Database accession no. 6796831
- DATABASE GNPD [online] MINTEL; 9 February 2021 (2021-02-09), ANONYMOUS: "Oat Milk Lemon Shampoo Powder", XP055776015, retrieved from https://www.gnpd.com/sinatra/recordpage/8476 695/ Database accession no. 8476695
- "Gentle Foaming Cleanser Combination or Oily Skin", GNPD, MINTEL, 1041089, 1 January 2009 (2009-01-01), XP002672807
- DATABASE GNPD [online] MINTEL; 4 June 2019 (2019-06-04), ANONYMOUS: "Deep Clear Washing Powder", XP055775906, retrieved from https://www.gnpd.com/sinatra/recordpage/6608 263/ Database accession no. 6608263
- DATABASE GNPD [online] MINTEL; 18 March 2019 (2019-03-18), ANONYMOUS: "Facial Washing Powder", XP055775941, retrieved from https://www.gnpd.com/sinatra/recordpage/6408 197/ Database accession no. 6408197
- DATABASE GNPD [online] MINTEL; 2 September 2016 (2016-09-02), ANONYMOUS: "Amazonian Ritual Powder Shampoo", XP055706987, retrieved from https://www.gnpd.com/sinatra/recordpage/4251 593/ Database accession no. 4251593
- DATABASE GNPD [online] MINTEL; 18 July 2017 (2017-07-18), ANONYMOUS: "Clay-Mud Cleansing Powder", XP055775879, retrieved from https://www.gnpd.com/sinatra/recordpage/4939 273/ Database accession no. 4939273
- DATABASE GNPD [online] MINTEL; 4 April 2020 (2020-04-04), ANONYMOUS: "Shower Powder with White Oyster Shell", XP055775892, retrieved from https://www.gnpd.com/sinatra/recordpage/7434 751/ Database accession no. 7434751
- DATABASE GNPD [online] MINTEL; 1 April 2020 (2020-04-01), ANONYMOUS: "Macadamia Orange Shampoo Powder", XP055776108, retrieved from https://www.gnpd.com/sinatra/recordpage/7504 051/ Database accession no. 7504051
- DATABASE GNPD [online] MINTEL; 15 April 2020 (2020-04-15), ANONYMOUS: "Powder Wash", XP055776065, retrieved from https://www.gnpd.com/sinatra/recordpage/7538 435/ Database accession no. 7538435

Remarques:

Le dossier contient des informations techniques présentées postérieurement au dépôt de la demande et ne figurant pas dans le présent fascicule.

**Description**

La présente invention concerne de nouvelles compositions cosmétiques et leur utilisation, notamment comme shampoing ou nettoyant. La présente invention concerne également un dispositif comprenant les nouvelles compositions.

**CONTEXTE DE L'INVENTION**

La plupart des produits d'hygiène personnel, tels que les shampoings ou les nettoyants sont commercialisés sous forme liquide. Ces produits comprennent majoritairement de l'eau, parfois jusqu'à 90% en masse par rapport au poids total du produit. La présence de quantités importantes d'eau a un impact négatif sur plusieurs points, par exemple sur l'environnement ou sur les exigences liées au transport du produit. En effet, le volume élevé du conditionnement conduit à un coût élevé de transport par le distributeur et des contraintes pour le consommateur, par exemple lors d'un voyage.

Compte tenu de la présence d'eau, les compositions cosmétiques sous forme liquide nécessitent des conservateurs, et possèdent des quantités de substances actives en pourcentage réduit. De ce fait, le consommateur dépense une somme importante pour peu de substances actives.

Depuis quelques années, quelques shampoings et nettoyants sous forme de poudre sont apparus sur le marché. Or, la plupart de ces produits contiennent des ingrédients chimiques synthétiques, potentiellement irritants et/ou des allergènes. De plus, la diversité de types de cheveux et de peau fait que la gamme de produits disponibles ne satisfait pas aux besoins spécifiques de chaque consommateur.

KR101308959 B1 divulgue un nettoyant au poudre comprenant un agent d'adhésion des poudres (l'amidon de maïs), du mannitol et au moins un tensioactif.

"Gentle Foaming Cleanser Combination or Oily Skin", Banque de données GNPD, abrégé no.1041089, 01.01.2009 montre un nettoyant comprenant entre autres ingrédients de l'α-glucan oligosaccharide et du mannitol.

GB2498543 A divulgue un poudre pour la peau contenant un prébiotique tel que l'α-glucan oligosaccharide (voir revendications).

"Deep Clear Washing Powder", Banque de données GNPD, abrégé no.6608263, 04.06.2019 montre un nettoyant sous forme de poudre comprenant un agent d'adhésion des poudres (l'amidon de maïs), du mannitol, de l'α-glucan oligosaccharide, et au moins un tensioactif.

"Facial Washing Powder", Banque de données GNPD, abrégé no.6408197, 18.03.2019 montre un nettoyant sous forme de poudre comprenant un agent d'adhésion des poudres (l'amidon de maïs), du mannitol, de l'α-glucan oligosaccharide et au moins un tensioactif.

"Amazonian Ritual Powder Shampoo", Banque de données GNPD, abrégé no.4251593, 02.09.2016 montre un shampooing sous forme de poudre comprenant du bentonite, du mannitol, au moins une huile et au moins un tensioactif.

Il existe donc un besoin de mettre à disposition des compositions alternatives, comprenant des ingrédients naturels ou d'origine naturelle, non-allergènes, sains, et dépourvus de conservateurs, de préférence ayant une activité protectrice de la flore cutanée.

L'un des buts de l'invention est de fournir une nouvelle association sous forme de poudre, comprenant un prébiotique.

Un autre but de l'invention est une nouvelle composition cosmétique sous forme de poudre comprenant un prébiotique.

Un troisième but de l'invention est de pouvoir utiliser la nouvelle association, ou une composition la comprenant comme shampoing ou nettoyant.

Un quatrième but est de fournir un dispositif comprenant un shampoing ou nettoyant sous forme de poudre, facile d'utilisation.

L'un des buts est de fournir une composition cosmétique dépourvue de conservateurs.

L'un des autres buts est de fournir une composition cosmétique dans laquelle plus de 15% en masse par rapport au poids total de la composition, notamment plus de 17%, est constitué de constituants et d'ingrédients ayant une propriété bénéfique pour la peau.

Les inventeurs ont entamé une recherche approfondie afin de mettre au point des shampoings et des nettoyants en poudre, ayant des propriétés sensorielles intéressantes, notamment par rapport au pouvoir moussant et à la rinçabilité. Les poudres mises au point sont fines et douces, et produisent une sensation de douceur lors de la manipulation

Un premier objet de la présente invention est une association comprenant les 4 constituants suivants :

- un agent d'adhésion des poudres,
- de l'α-glucan oligosaccharide,
- au moins une huile, et

- au moins un tensioactif,

ladite association étant sous forme de poudre substantiellement dépourvue d'eau,
ledit agent d'adhésion, ledit α-glucan oligosaccharide, et ledit au moins un tensioactif étant sous forme de poudre,
ladite au moins une huile étant notamment sous forme de poudre,
dans laquelle ladite association comprend optionnellement en outre au moins un ingrédient.

La somme des constituants et ingrédients sous forme de poudre représente notamment de 90% à 100% en masse par rapport au poids total de l'association selon la présente invention. Par rapport à la somme des constituants et ingrédients, on entend également par « de 90 à 100% » les gammes suivantes : de 92 à 100%, de 94 à 100%, de 96 à 100%, de 98 à 100%, de 90 à 98%, de 90 à 96%, de 90 à 94%, de 90 à 92%, de 91 à 99%, de 92 à 98%, de 93 à 97%, de 94 à 96%.

Selon un mode de réalisation particulier, la présente invention concerne une association telle que définie ci-dessus, comprenant les 5 constituants suivants :

- un agent d'adhésion des poudres,
- du mannitol,
- de l'α-glucan oligosaccharide,
- au moins une huile, et
- au moins un tensioactif,

dans laquelle le pourcentage massique d'eau par rapport à la masse totale de l'association est compris de 0 à 10%, notamment de 0,1 à 10%, en particulier de 0,1 à 5%,
ladite association étant sous forme de poudre,
ledit agent d'adhésion, ledit mannitol, ledit α-glucan oligosaccharide, et ledit au moins un tensioactif étant sous forme de poudre, ladite au moins une huile étant notamment sous forme de poudre,
dans laquelle ladite association comprend en outre au moins un ingrédient,
le poids total dudit au moins un ingrédient est notamment compris de 0 à 15% en masse par rapport au poids total de l'association, en particulier de 0 à 10%, ou de 2 à 15%.

Selon un mode de réalisation particulier, la présente invention concerne une association telle que définie ci-dessus, dans laquelle

le pourcentage massique d'eau par rapport à la masse totale de l'association est compris de 0% à 1%, notamment de 0 à 0,5%, et
le poids total dudit au moins un ingrédient est compris de 2 à 15% en masse par rapport au poids total de l'association.

Selon un mode de réalisation particulier, la présente invention concerne une association telle que définie ci-dessus, dans laquelle

le pourcentage massique d'eau par rapport à la masse totale de l'association est de compris de 0,1 à 10%, notamment d'au moins 1 % à 10%, et
le poids total dudit au moins un ingrédient est de 0% en masse par rapport au poids total de l'association.

Selon un mode de réalisation particulier, la présente invention concerne une association telle que définie ci-dessus, dans laquelle

le pourcentage massique d'eau par rapport à la masse totale de l'association est compris de 0% à 1%, notamment de 0 à 0,5%, et
le poids total dudit au moins un ingrédient est de 0% en masse par rapport au poids total de l'association.

Selon un mode de réalisation particulier, la présente invention concerne une association telle que définie ci-dessus, dans laquelle

le pourcentage massique d'eau par rapport à la masse totale de l'association est de 0,1 à 10%, notamment d'au moins 1 à 10%, et
le poids total dudit au moins un ingrédient est compris de 2 à 15% en masse par rapport au poids total de l'association.

Selon la présente invention, on entend par « *constituants* » les constituants énoncés ci-dessus, à savoir : un agent d'adhésion des poudres, du mannitol, de l'α-glucan oligosaccharide, au moins une huile, et au moins un tensioactif. Au moins 3 des 5 constituants ont une activité sur la peau.

Selon la présente invention, on entend par « *ingrédient* » les composés autres que les constituants pouvant être optionnellement contenus. Les ingrédients ont soit une activité sur la peau, soit des propriétés au niveau de la sensorialité.

L'association selon la présente invention est constituée de constituants et d'ingrédients naturels, ou d'origine naturelle, et d'origine non-animale.

L'expression « *association* » est synonyme de « *combinaison* ».

Par « *agent d'adhésion des poudres* » on entend, au sens de la présente invention, un support qui permet une bonne adhésion des poudres. Il s'agit d'un agent permettant l'adhésion des différents constituants et ingrédients de la poudre, et de modifier la rhéologie de l'association lors de son utilisation. Il s'agit plus particulièrement d'additifs modificateurs de viscosité permettant de modifier la texture de l'association lors de son utilisation. Les agents d'adhésion des poudres utilisable dans la présente invention sont notamment des biopolymères à base de glucides.

Par « *α-glucan oligosaccharide* » on entend un oligosaccharide constitué d'un enchaînement d'unités de D-glucose liés entre eux par des liaisons glycosidiques de forme α. Les α-glucan oligosaccharides sont synthétisés par une réaction de transglucosylation catalysée par des enzymes de la famille des glucanes-saccharases. L'α-glucan oligosaccharide utilisable dans le cadre de la présente invention est un « *prébiotique* ». Les prébiotiques servent à maintenir un milieu favorable au développement de la flore endogène cutanée. Les prébiotiques sont en particulier utilisés pour orienter le métabolisme de l'ensemble de la flore cutanée afin de maintenir un bon équilibre de cette dernière, tout en ne favorisant pas le développement de certaines bactéries pathogènes.

Dans le cadre de la présente invention l'α-glucan oligosaccharide ayant des propriétés prébiotiques est de préférence constitué de 2 à 50 unités de sucre, notamment de 2 à 10 unités de sucre.

Parmi les oligosaccharides utilisables et disponibles dans le commerce, on peut citer le Bioecolia® (INCI : α-glucan oligosaccharide) ou l'Ecoskin® (INCI : Alpha-glucan oligosaccharide, Polymnia sonchifolia root juice, Maltodextrin, Lactobacillus) de la société Solabia, ou Biolin/P (INCI : Inulin (and) Alpha-Glucan Oligosaccharide) de la société Gobiotics.

L'association selon la présente invention comprend de préférence 1 ou 2 huiles.

L'association selon la présente invention comprend de préférence 1 ou 2 tensioactifs.

Par « *substantiellement dépourvue d'eau* » on entend que le pourcentage massique d'eau par rapport à la masse totale de l'association est compris de 0 à 10%, notamment de 0 à 5%, notamment de 0 à 3%, notamment de 0 à 2%, notamment de 0 à 1%, en particulier de 0 à 0,5%.

L'association sous forme de poudre, ainsi que l'agent d'adhésion, le mannitol, l'α-glucan oligosaccharide, et l'au moins un tensioactif sont solides à température ambiante.

L'au moins une huile est notamment solide à température ambiante.

Par « *température ambiante* » on entend, selon la présente invention, une température comprise de 20 à 25 °C.

L'au moins un ingrédient est en particulier un principe-actif, notamment choisi parmi :

l'acide de fleur,
le Zinc PCA,
un pigment,
le charbon,
un antioxydant, en particulier la vitamine C, ou un de ses dérivés,
du thé en poudre, notamment du thé vert matcha, du thé fermenté, du thé kombutcha en poudre, la centella asiatica, ou du madécassoside,
des sucres, notamment le xylitol, le rhamnose, ou les fructooligosaccharides,
l'aloe vera,
une protéine, notamment la protéine de pois, la protéine de soja, la protéine de blé, ou la protéine de riz,
le lait d'amande douce, ou un ou plusieurs de ses composants, notamment le prunus dulcis, le sirop de riz ou la fibre d'acacia,
un parfum, notamment un parfum abricot amande,
le ghassoul,
l'argile blanche, notamment le kaolin,
l'hyaluronate de sodium,
l'acide hyaluronique,
l'acide lactique,
le macérât de vanille (INCI : Corylus avellana Seed Oil/Vanilla planifolia fruit),
la maltodextrine,

la poudre de bambou,

la spiruline,

la phycocianine,

des champignons fermentés,

du guarana,

de la caféine,

de l'acérola,

la camomille,

les poudres ayurvédiques,

les vitamines, notamment la vitamine B3, ou la vitamine B5,

les glycosphingolipides et glycolipides, par exemple de la marque Ceramosides™ HP (INCI : Glycosphingolipides, glycolipides),

les exopolysaccharides, et

les postbiotiques, notamment le lactobacilus ferment comme par exemple de la marque lactobiotyl® (INCI : Maltodextrin and Lactobacillus Ferment),

la pyroctolamine,

la piroctone olamine,

un extrait de graines de camélia (INCI : Camellia oleifera seed extract),

un extrait de bleuet (INCI : Centaurea cyanus flower extract),

un extrait de fleur de camomille (INCI Maltodextrin, Chamomilla Recutita Flower Extract),

ou un mélange de ceux-ci,

ledit au moins un ingrédient est notamment solide à température ambiante.

Le poids total dudit au moins un ingrédient est notamment compris de 0 à 15% en masse par rapport au poids total de l'association, en particulier de 0 à 10%, ou de 2 à 10%.

Le « Zinc PCA » est un sel de zinc de l'acide pyrrolidone carboxylique, qui est commercialisé par exemple sous le nom de Zincidone®.

Parmi les pigments, on peut citer l'Unicert violet K7025-J (INCI : CI 60730), comprenant le colorant acide benzène sulfonique 2-[(9,10-dihydro-4-hydroxy-9,10-dioxo-1-anthracényl) amino]-5-méthyl monosel de sodium (CAS 4430-18-6)

Parmi les exopolysaccharides on peut citer :

le saccharide isomerate, par exemple

de la marque Epidermist™ 4.0 (INCI : Aqua, Saccharide Isomerate),

de la marque EPS Seamat™ (INCI : Aqua, Saccharide Isomerate),

de la marque EPS seapur® (INCI : Water, Phenethyl alcohol, Saccharide isomerate),

de la marque EPS seaglow® (INCI : Water, Saccharide isomerate, Sodium benzoate, Citric acid)

de la marque l'Actibiome de la société Codif (INCI : Glycerin, Water, Seawater, Laminaria digitata extract, Chlorella vulgaris extract, saccharide isomerate, phenethylalcohol),

l'Alteromonas ferment extract, par exemple de la marque EPS Seafill™ (INCI : Aqua, Alteromonas Ferment Extract).

ou les exopolysaccharides de la marque, EPS seawhite®.

Parmi les poudres ayurvédiques on peut citer :

la poudre de Shikakaï ( INCI : Acacia concinna fruit powder),

la poudre de Reetha (INCI : Sapindus mukorossi powder),

le sidr (INCI : Zizyphus jujuba leaf powder),

la poudre de brahmi (INCI : Bacopa Monnieri),

la Poudre de Bhringaraj (INCI : Eclipta alba powder),

l'amla (INCI : Emblica officinalis fruit powder),

la poudre de Fenugrec (methi) (INCI : Trigonella foenum-graecum seed powder),

la poudre d'Hibiscus (INCI : Hibiscus sabdariffa flower powder),

la Kapoor Kachli (INCI : Hedychium Spicatum),

la poudre de Tulsi (INCI : Ocimum sanctum leaf powder),

la poudre de kachur Sugandhi (INCI : Kaempferia galanga root powder),

la poudre de Neem (INCI : Azadirachta indica leaves powder),

la poudre de Nagarmotha (INCI : Cyperus rotundus root powder), et

la poudre d'orange (INCI : Citrus aurantium peel powder).

Comme « *antioxydant* », on peut citer, par exemple le tocophérol (vitamine E) ou l'acide ascorbique (vitamine C), ou un dérivé de la vitamine C, notamment l'ascorbyl glucoside.

Les parfums utilisés dans la présente invention sont des parfums naturels ou d'origine naturelle. Il s'agit notamment d'un parfum herbes et thé, d'un parfum abricot amande, ou d'un parfum lait de coco, qui sont par exemple commercialisés par la société Natflor.

L'association selon la présente invention est notamment dépourvue :

d'agents conservateurs, tel que le phenyléthanol,
de parabènes,
de polyéthylèneglycol (PEG),
de sulfates,
de talc,
de silicones,
d'allergènes,
de parfums synthétiques, et
d'actifs irritants.

L'association selon la présente invention est en particulier dépourvue de sels minéraux de métaux lourds.

L'association selon la présente invention est en particulier dépourvue de matières non-naturelles, ou d'origine non-naturelle, et d'origine animale.

Par l'expression « *dépourvue* » on entend des quantités massiques inférieures à 100 ppm, notamment inférieures à 50 ppm, en particulier inférieures à 10 ppm.

Parmi les agents conservateurs un peut citer, à titre d'exemple, le triclosan, le triclocarban, le phenyléthanol, le benzoate de benzyle, methylisothiazolinone, zinc pyrithione, le chlorure de benzalkonium, et alcool benzylique. L'association selon la présente invention est dépourvue de tout conservateur chimique, non-naturel.

Parmi les parabènes un peut citer, à titre d'exemple le parabène de méthyle, le parabène de propyle, le parabène de butyle, et le parabène d'éthyle.

Par « *polyéthylèneglycol* » on entend un polymère constitué de monomères d'éthylène glycol pouvant présenter des poids moléculaires divers, on peut citer, à titre d'exemple le PEG-1000 et le PEG-2000.

Parmi les silicones on peut citer, à titre d'exemple, le diméthicone, le cyclométhicone, l'amodiméthicone, le ceteraryl méthicone, le diméthiconol, et le stearyl diméthicone.

Par « *allergènes* » on entend des substances connues pour être irritant ou allergène. A titre d'exemple on peut citer : l'α-Isomethyl ionone, le cinnamal d'amyle, l'alcool d'amylcinnamyle, l'alcool anisique, l'alcool benzylique, le benzoate de benzyle, le cinnamate de benzyle, le salicylate de benzyle, le methylpropional de butylphenyl, le cinnamal, l'alcool de cinnamyle, le citral, le citronellol, la coumarine, l'eugénol, le farnésol, le géraniol, le cinnamal d'hexyle, l'ydroxycitronnellal, l'isoeugénol, le limonène, le linalool, et l'octynoate de 2-méthyle.

Par « *actifs irritants* » on entend des produits présentant un risque d'irritation de la peau. Il s'agit de tout actif connu pour avoir une propriété irritante dans le domaine de la cosmétique. On peut citer à titre d'exemple :

les tensioactifs sulfatés, tels que le sodium lauryl sulfate ou le sodium coco sulfate, et
le guar hydroxypropyltrimoniumchloride.

Selon un autre mode de réalisation particulier, la présente invention concerne une association consistant en les 5 constituants suivants :

- un agent d'adhésion des poudres,
- du mannitol,
- de l'α-glucan oligosaccharide,
- au moins une huile, et
- au moins un tensioactif,

ladite association étant sous forme de poudre substantiellement dépourvue d'eau,
ledit agent d'adhésion, ledit mannitol, ledit α-glucan oligosaccharide, et ledit au moins un tensioactif étant sous forme de poudre, ladite au moins une huile étant notamment sous forme de poudre.

Selon un autre mode de réalisation particulier, la présente invention concerne une association consistant en les 5 constituants suivants :

- un agent d'adhésion des poudres,
- du mannitol,
- de l'α-glucan oligosaccharide,
- au moins une huile, et
- au moins un tensioactif,

ladite association étant sous forme de poudre,
dans laquelle le pourcentage massique d'eau est compris de 0 à 10% par rapport à la masse totale de l'association,
ledit agent d'adhésion, ledit mannitol, ledit α-glucan oligosaccharide, et ledit au moins un tensioactif étant sous forme de poudre, ladite au moins une huile étant notamment sous forme de poudre.

Dans ce mode de réalisation particulier, l'association est dépourvue d'ingrédients autres que les 5 constituants indiqués ci-dessus.

Selon un autre mode de réalisation particulier, la présente invention concerne une association telle que définie ci-dessus, dans laquelle la densité de la poudre est comprise de 0,35 à 0,55 g/ml, notamment de 0,4 à 0,5 g/ml.

Au sens de la présente invention, la densité correspond à la densité « non-tassée ».

Selon un autre mode de réalisation particulier, la présente invention concerne une association telle que définie ci-dessus, dans laquelle l'agent d'adhésion des poudres est choisi parmi :

- l'amidon de maïs,
- la poudre d'acacia,
- la poudre de maltodextrine,
- la poudre d'extrait de pomme, notamment de la marque Vitacell CS5 Apple de JRS Rettenmaier (INCI Pyrus Malus (Apple) Fiber),
- la poudre de tapioca, notamment le Jojoba Silk Like Powder de Jojoba desert (INCI: *Simmondsia Chinesis* (Jojoba) Seed Oil, Tapioca Pure)
- la poudre de riz,
- l'extrait de son de riz, et
- la poudre de Marante

ou un mélange de ceux-ci.

Selon un autre mode de réalisation particulier, la présente invention concerne une association telle que définie ci-dessus, dans laquelle l'huile est choisie parmi :

l'huile de jojoba,
l'huile d'amande douce,
l'huile d'amande amère,
l'huile d'argan,
l'huile de sésame,
l'huile de camélia,
l'huile de carthame,
l'huile de moringa,
l'huile de ricin,
l'huile de coco,
l'huile d'abricot,
l'huile de lin,
l'huile de grenade,
l'huile de pépins de raisin,
les huiles de figues de barbarie,
l'huile de marula,
l'huile d'onagre,
l'huile d'avocat,
l'huile de framboise,
l'huile de bourrache,
l'huile d'olive,
l'huile de neem,
l'huile de tournesol,
l'huile de noix ou de noisette,

l'huile d'amla,
l'huile de prune,
l'huile de café,
l'huile de chanvre,
un macérat de vanille,
une poudre d'huile, et
une huile fermentée,
ou un mélange de celles-ci.

Par « *poudre d'huile* » on entend des huiles initialement liquides à température ambiante, qui ont été formulées pour être solides à température ambiante. Les huiles peuvent par exemple être encapsulées pour conduire à des microcapsules sous forme de poudre. Parmi des agents d'encapsulation on peut citer l'alginate, la maltodextrine, la poudre de marante, ou l'amidon de maïs.

Parmi les huiles susceptibles d'être formulées sous forme de poudre, on peut citer les huiles végétales, notamment les huiles telles que définies ci-dessus.

On peut notamment citer de la poudre d'huile d'argan (l'huile d'argan enrobée par de la maltodextrine), de la poudre de jojoba, de la poudre d'huile de ricin, de la poudre d'huile d'amande douce, ou la poudre d'huile de coco.

Par « *huile fermentée* » on entend une huile ayant subi un procédé de fermentation. Parmi les huiles susceptibles d'être fermentées, on peut citer les huiles végétales, notamment les huiles telles que définies ci-dessus. On peut notamment citer l'huile de jojoba fermentée, l'huile d'argan fermentée, l'huile de moringa fermentée, l'huile de sésame fermentée, l'huile d'amande douce fermentée, l'huile de chanvre fermentée, l'huile de carthame fermentée, l'huile de camélia fermentée, ou l'huile d'olive fermentée.

Selon un autre mode de réalisation particulier, la présente invention concerne une association telle que définie ci-dessus, dans laquelle l'huile est une poudre d'huile telle que définie ci-dessus.

Selon un autre mode de réalisation particulier, la présente invention concerne une association telle que définie ci-dessus, dans laquelle l'huile est une huile fermentée telle que définie ci-dessus.

Selon un autre mode de réalisation particulier, la présente invention concerne une association telle que définie ci-dessus, dans laquelle le tensioactif est choisi parmi :
lauryl glucoside, decyl glucoside, caprylyl/capryl glucoside, coco-glucoside, hexyl glucoside, sodium cocoyl glutamate, disodium cocoyl glutamate, sodium stearoyl glutamate, sodium lauroyl glutamate, sodium myristoyl glutamate, coco-betaine, cocamidopropyl betaine, disodium cocoamphodiacetate, sodium arganamphoacetate, sodium babassuamphoacetate, sodium cocoabutteramphoacetate, sodium cottonseedamphoacetate, sodium sheabutteramphoacetate, sodium mangoamphoacetate, sodium olivamphoacetate, sodium ricebranamphoacetate, sodium sweetalmondamphoacetate, sodium sunflowerseedamphoacetate, potassium cocoate, potassium palmate, potassium laurate, potassium olivate, sodium coco-sulfate, sodium cocoamphoacetate, disodium coco-glucoside citrate, sodium coco-glucoside tartrate, sodium lauroamphoacetate, cocoyl methyl glucamide, capryloyl/caproyl methyl glucamide, lauroyl/myristoyl methyl glucamide, ammonium lauryl sulfate, ammonium coco-sulfate, magnésium lauryl sulfate, sodium C12-18 alkyl sulfate, zinc coco-sulfate, sodium cocoyl alaninate, sodium lauryl sulfate, sodium cetearyl sulfate, sodium cocoyl isethionate, sodium methyl oleoyl taurate, sodium methyl cocoyl taurate, cocamide MEA, cocamide MIPA, Sodium C14-16 olefin sulfonate, sodium lauryl sulfoacetate, sodium lauroyl sarcosinate, sodium laureth sulfate, ammonium laureth sulfate, cocamidopropyl hydroxysultaine, disodium laureth sulfosuccinate, disodium lauryl sulfosuccinate, sodium myreth sulfate, laureth-5 acide carboxylique et la saponine.

Selon un autre mode de réalisation particulier, la présente invention concerne une association telle que définie ci-dessus, dans laquelle le tensioactif est un tensioactif anionique, notamment choisi parmi :

le cocoyl glutamate de sodium, et
le cocoyl iséthionate de sodium,
ou un mélange de ceux-ci.

Selon un autre mode de réalisation particulier, la présente invention concerne une association telle que définie ci-dessus, dans laquelle la quantité totale d'agent d'adhésion des poudres est comprise de 5 à 85%, notamment de 5 à 15%, ou de 70 à 85%, en pourcentage en poids par rapport au poids total de l'association.

Par rapport à la quantité totale d'agent d'adhésion, on entend également par « de 5 à 85% » les gammes suivantes : de 5 à 80%, de 5 à 70%, de 5 à 60%, de 5 à 50%, de 5 à 40%, de 5 à 30%, de 5 à 20%, de 5 à 15%, de 10 à 85%, de 20 à 85%, de 30 à 85%, de 40 à 85%, de 50 à 85%, de 50 à 85%, de 60 à 85%, de 70 à 85%, de 80 à 85%, de 10 à 80%, de 20 à 70%, de 30 à 60%, de 40 à 50%.

Selon un autre mode de réalisation particulier, la présente invention concerne une association telle que définie ci-dessus, dans laquelle la quantité totale de mannitol est comprise de 5 à 20%, notamment de 5 à 15% en pourcentage en poids par rapport au poids total de l'association.

Selon un autre mode de réalisation particulier, la présente invention concerne une association telle que définie ci-dessus, dans laquelle la quantité totale d'α-glucan oligosaccharide est comprise de 0,5 à 2%, notamment d'environ 1 % en pourcentage en poids par rapport au poids total de l'association.

Par rapport à la quantité totale d'α-glucan oligosaccharide, on entend également par « de 0,5 à 2% » les gammes suivantes : de 0,6 à 2%, de 0,8 à 2%, de 1 à 2%, de 1,2 à 2%, de 1,4 à 2%, de 1,6 à 2%, de 1,8 à 2%, de 0,5 à 1,8%, de 0,5 à 1,6%, de 0,5 à 1,4%, de 0,5 à 1,2%, de 0,5 à 1,0%, de 0,5 à 0,8%, de 0,6 à 1,8%, de 0,7 à 1,6%, de 0,8 à 1,4%, de 0,9 à 1,2%.

Selon un autre mode de réalisation particulier, la présente invention concerne une association telle que définie ci-dessus, dans laquelle la quantité totale d'huile est comprise de 1 à 50%, notamment de 1 à 10%, notamment de 2 à 8% en pourcentage en poids par rapport au poids total de l'association.

Par rapport à la quantité totale d'huile, on entend également par « de 1 à 50% » les gammes suivantes : de 5 à 50%, de 10 à 50%, de 20 à 50%, de 30 à 50%, de 40 à 50%, 1 à 40%, de 1 à 30%, de 1 à 20%, de 1 à 10%, de 5à 40%, de 10 à 30%, de 15 à 20%.

Selon un autre mode de réalisation particulier, la présente invention concerne une association telle que définie ci-dessus, dans laquelle la quantité totale de tensioactif est comprise de 1 à 80%, notamment de 2 à 20%, ou de 40 à 80% en pourcentage en poids par rapport au poids total de l'association.

Par rapport à la quantité totale de tensioactif, on entend également par « de 1 à 80% » les gammes suivantes : de 10 à 80%, de 20 à 80%, de 30 à 80%, de 40 à 80%, de 50 à 80%, de 60 à 80%, de 70 à 80%, de 1 à 70%, de 1 à 60%, de 1 à 50%, de 1 à 40%, de 1 à 30%, de 1 à 20%, de 1 à 10%, de 5 à 10%, de 10 à 70%, de 20 à 60%, de 30 à 50%.

Selon un autre mode de réalisation particulier, la présente invention concerne une association telle que définie ci-dessus, ladite association étant sous la forme :

- d'un shampoing, ledit shampoing comprenant notamment :
  deux tensioactifs en quantité totale comprise de 40 à 80%, notamment de 70 à 80%, en pourcentage en poids par rapport au poids total de la composition, et un agent d'adhésion des poudres, en quantité comprise de 5 à 15% en pourcentage en poids par rapport au poids total de la composition,
  ou
- d'un nettoyant, ledit nettoyant comprenant notamment :

  un tensioactif en quantité comprise de 2 à 20%, notamment de 5 à 10% en pourcentage en poids par rapport au poids total de la composition, et
  un agent d'adhésion des poudres, en quantité comprise de 70 à 85% en pourcentage en poids par rapport au poids total de la composition.

La présente Invention concerne également une association consistant en les 4 constituants suivants :

- un agent d'adhésion des poudres,
- de l'α-glucan oligosaccharide,
- au moins une huile, et
- au moins un tensioactif,

  ladite association étant sous forme de poudre,
  dans laquelle le pourcentage massique d'eau est compris de 0 à 10% par rapport à la masse totale de l'association, ledit agent d'adhésion, ledit α-glucan oligosaccharide, et ledit au moins un tensioactif étant sous forme de poudre, ladite au moins une huile étant notamment sous forme de poudre.

Selon ce mode de réalisation, les 4 constituants sont tels que définis ci-dessus.

Un autre objet de la présente invention est l'utilisation d'une association telle que définie ci-dessus, dans une composition cosmétique.

Un autre objet de la présente invention est une composition cosmétique, comprenant ou constitué par l'association telle que définie ci-dessus, dans un milieu cosmétiquement acceptable, ladite composition étant sous la forme d'une poudre substantiellement dépourvue d'eau.

Par « *un milieu cosmétiquement acceptable* », on entend au sens de l'invention un milieu compatible avec une utilisation en cosmétique.

Selon un mode de réalisation particulier, la présente invention concerne une composition cosmétique telle que définie ci-dessus, comprenant une association telle que définie ci-dessus, dans un milieu cosmétiquement acceptable,

ladite composition étant sous la forme d'une poudre,
dans laquelle le pourcentage massique d'eau est compris de 0 à 10% par rapport à la masse totale de la composition.

Selon un mode de réalisation particulier, la présente invention concerne une composition cosmétique, comprenant une association telle que définie ci-dessus, dans un milieu cosmétiquement acceptable,

ladite composition étant sous la forme d'une poudre,
dans laquelle le pourcentage massique d'eau est compris de 0 à 10% par rapport à la masse totale de la composition,
dans laquelle ladite composition comprend en outre un pigment ou de la piroctone olamine.

Selon ce mode de réalisation, l'association comprise dans la composition cosmétique est dépourvue de mannitol.

Selon un mode de réalisation particulier, la présente invention concerne une composition cosmétique telle que définie ci-dessus, comprenant les 5 constituants suivants :

- un agent d'adhésion des poudres,
- du mannitol,
- de l'α-glucan oligosaccharide,
- au moins une huile, et
- au moins un tensioactif,

dans laquelle le pourcentage massique d'eau par rapport à la masse totale de la composition cosmétique est compris de 0 à 10%, notamment de 0,1 à 10%, en particulier de 0,1 à 5%, ladite composition cosmétique étant sous forme de poudre,
ledit agent d'adhésion, ledit mannitol, ledit α-glucan oligosaccharide, et ledit au moins un tensioactif étant sous forme de poudre, ladite au moins une huile étant notamment sous forme de poudre,
dans laquelle ladite composition cosmétique comprend en outre au moins un ingrédient,
le poids total dudit au moins un ingrédient est notamment compris de 0 à 15% en masse par rapport au poids total de la composition, en particulier de 0 à 10%, ou de 2 à 15%.

Selon un autre mode de réalisation particulier, la présente invention concerne une composition cosmétique telle que définie ci-dessus, dans laquelle la densité de la poudre est comprise de 0,35 à 0,55 g/ml, notamment de 0,4 à 0,5 g/ml.

Selon un autre mode de réalisation particulier, la présente invention concerne une composition cosmétique telle que définie ci-dessus, ladite association étant présente à raison de 70 à 100%, notamment de 85 à 100% en pourcentage en poids par rapport au poids total de la composition.

Selon un autre mode de réalisation particulier, la présente invention concerne une composition cosmétique telle que définie ci-dessus, dans laquelle la quantité totale d'agent d'adhésion des poudres est comprise de 3,5 à85%, notamment de 3,5 à 15%, ou de 50 à 85%, en pourcentage en poids par rapport au poids total de la composition.

Par rapport à la quantité totale d'agent d'adhésion des poudres, on entend également par « de 3,5 à 85% » les gammes suivantes : de 3,5 à 80%, de 3,5 à 70%, de 3,5 à 60%, de 3,5 à 50%, de 3,5 à 40%, de 3,5 à 30%, de 3,5 à 20%, de 3,5 à 15%, de 10 à 85%, de 20 à 85%, de 30 à 85%, de 40 à 85%, de 50 à 85%, de 50 à 85%, de 60 à 85%, de 70 à 85%, de 80 à 85%, de 10 à 80%, de 20 à 70%, de 30 à 60%, de 40 à 50%.

Selon un autre mode de réalisation particulier, la présente invention concerne une composition cosmétique telle que définie ci-dessus, dans laquelle la quantité totale de mannitol est comprise de 3,5 à 20%, notamment de 8 à 15%, en pourcentage en poids par rapport au poids total de la composition.

Selon un autre mode de réalisation particulier, la présente invention concerne une composition cosmétique telle que définie ci-dessus, dans laquelle la quantité totale d'α-glucan oligosaccharide est comprise de 0.35 à 2%, notamment d'environ 1%, en pourcentage en poids par rapport au poids total de la composition.

Par rapport à la quantité totale d'α-glucan oligosaccharide, on entend également par « de 0,35 à 2% » les gammes suivantes : de 0,4 à 2%, de 0,6 à 2%, de 0,8 à 2%, de 1 à 2%, de 1,2 à 2%, de 1,4 à 2%, de 1,6 à 2%, de 1,8 à 2%, de 0,35 à 1,8%, de 0,35 à 1,6%, de 0,35 à 1,4%, de 0,35 à 1,2%, de 0,35 à 1,0%, de 0,35 à 0,8%, de 0,35 à 0,5%, de 0,4 à 1,8%, de 0,6 à 1,6%, de 0,8 à 1,4%, de 1,0 à 1,2%.

Selon un autre mode de réalisation particulier, la présente invention concerne une composition cosmétique telle que définie ci-dessus, dans laquelle la quantité totale d'huile est comprise de 0,7 à 50% en pourcentage en poids par rapport au poids total de la composition.

Par rapport à la quantité totale d'huile, on entend également par « de 0,7 à 50% » les gammes suivantes : de 1 à 50%, de 5 à 50%, de 10 à 50%, de 20 à 50%, de 30 à 50%, de 40 à 50%, de 0,7 à 40%, de 0,7 à 30%, de 0,7 à 20%, de 0,7 à 10%, de 0,7 à 5%, de 5 à 40%, de 10 à 30%, de 15 à 20%.

Selon un autre mode de réalisation particulier, la présente invention concerne une composition cosmétique telle que définie ci-dessus, dans laquelle la quantité totale de tensioactif est comprise de 0,7 à 80%, notamment comprise de 1,4 à 20%, ou comprise de 30 à 80% en pourcentage en poids par rapport au poids total de la composition.

Par rapport à la quantité totale de tensioactif, on entend également par « de 0,7 à 80% » les gammes suivantes : de 10 à 80%, de 20 à 80%, de 30 à 80%, de 40 à 80%, de 50 à 80%, de 60 à 80%, de 70 à 80%, de 1 à 70%, de 1 à 60%, de 1 à 50%, de 1 à 40%, de 1 à 30%, de 1 à 20%, de 1 à 10%, de 10 à 70%, de 20 à 60%, de 30 à 50%.

Selon un autre mode de réalisation particulier, la présente invention concerne une composition cosmétique telle que définie ci-dessus, ladite composition étant dépourvue :

d'agents conservateurs, tel le phenyléthanol,
de parabènes,
de polyéthylèneglycol (PEG),
de sulfates,
de talc,
de silicones,
d'allergènes,
de parfums synthétiques, et
d'actifs irritants.

La composition cosmétique de la présente invention est en particulier dépourvue de sels minéraux de métaux lourds.

Lesdits agents conservateurs, sels minéraux de métaux lourds, parabènes, polyéthylèneglycol (PEG), sulfates, talc, silicones, allergènes, parfums synthétiques, et actifs irritants sont tels que définis précédemment.

Les compositions cosmétiques selon la présente invention sont en particulier dépourvues de matières non-naturelles ou d'origine non-naturelle, et de matières animales.

Les compositions cosmétiques selon la présente invention peuvent également comprendre au moins un ingrédient tel que défini ci-dessus.

Selon un mode de réalisation particulier, la présente invention concerne une composition cosmétique telle que définie ci-dessus, dans laquelle

la composition comprend en outre au moins un ingrédient, en particulier un principe-actif, notamment choisi parmi :

l'acide de fleur,
le Zinc PCA,
un pigment,
le charbon,
un antioxydant, en particulier la vitamine C, ou un de ses dérivés,
du thé en poudre, notamment du thé vert matcha, du thé fermenté, du thé kombutcha en poudre, la centella asiatica, ou du madécassoside,
des sucres, notamment le xylitol, le rhamnose, ou les fructooligosaccharides, l'aloe vera,
une protéine, notamment la protéine de pois, la protéine de soja, la protéine de blé, ou la protéine de riz,
le lait d'amande douce, ou un ou plusieurs de ses composants, notamment le prunus dulcis, le sirop de riz ou la fibre d'acacia,
un parfum, notamment un parfum abricot amande,
le ghassoul,
l'argile blanche, notamment le kaolin
l'hyaluronate de sodium,
l'acide hyaluronique,
l'acide lactique,
le macérât de vanille,
la maltodextrine,
la poudre de bambou,
la spiruline,
la phycocianine,
des champignons fermentés,
du guarana,
de la caféine,

de l'acérola,

la camomille,

les poudres ayurvédiques,

les vitamines, notamment la vitamine B3, ou la vitamine B5,

les glycosphingolipides et glycolipides, par exemple de la marque Ceramosides™ HP (INCI: Glycosphingolipids, glycolipids),

les exopolysaccharides,et

les postbiotiques, notamment le lactobacilus ferment, comme par exemple de la marque lactobiotyl® (INCI : Maltodextrin and Lactobacillus Ferment),

la pyroctolamine,

la piroctone olamine,

un extrait de graines de camélia (INCI : Camellia oleifera seed extract),

un extrait de bleuet (INCI : Centaurea cyanus flower extract),

un extrait de fleur de camomille (INCI Maltodextrin, Chamomilla Recutita Flower Extract),

ou un mélange de ceux-ci,

ledit au moins un ingrédient est notamment solide à température ambiante.

Selon un autre mode de réalisation particulier, la présente invention concerne une composition cosmétique telle que définie ci-dessus, ladite composition étant sous la forme :

- d'un shampoing, ledit shampoing comprenant notamment :
  deux tensioactifs en quantité totale comprise de 30 à 80%, notamment de 50 à 80% en pourcentage en poids par rapport au poids total de la composition, et un agent d'adhésion des poudres, en quantité comprise de 3,5 à 15% en pourcentage en poids par rapport au poids total de la composition,
  ou

- d'un nettoyant, ledit nettoyant comprenant notamment :

  un tensioactif à raison de 1,4 à 20% en pourcentage en poids par rapport au poids total de la composition, et un agent d'adhésion des poudres, en quantité comprise de 50 à 85%, notamment de 70 à 85% en pourcentage en poids par rapport au poids total de la composition.

Selon un mode de réalisation préféré, la présente invention concerne une composition cosmétique telle que définie ci-dessus, dans laquelle ladite composition comprend, en pourcentage en poids par rapport au poids total de la composition :

- un agent d'adhésion des poudres, notamment l'amidon de maïs, à raison de 8 à 11%, et
- du mannitol à raison de 9 à 11%,
- de l'α-glucan oligosaccharide, à raison de 0,5 à 1,5%,
- de l'huile d'Argan, à raison de 3 à 5%,
- du cocoyl glutamate de sodium à raison de 25 à 35%,
- du cocoyl iséthionate de sodium à raison de 35 à 45%,
- de l'aloe vera à raison de 0,5 à 1%,
- de la protéine de pois à raison de 2 à 3%, et
- du parfum abricot amande à raison de 1 à 3%,

ladite composition étant notamment un shampoing.

Selon un autre mode de réalisation préféré, la présente invention concerne une composition cosmétique telle que définie ci-dessus, dans laquelle ladite composition comprend, en pourcentage en poids par rapport au poids total de la composition :

- un agent d'adhésion des poudres, notamment l'amidon de maïs, à raison de 10 à 12%,
- du mannitol à raison de 9 à 10%,
- de l'α-glucan oligosaccharide, à raison de 0,5 à 1,5%,
- du lait d'amande douce, à raison de 6 à 7%,
- du cocoyl glutamate de sodium à raison de 25 à 35%, et
- du cocoyl iséthionate de sodium à raison de 35 à 45%,

ladite composition étant notamment un shampoing.

Dans ce mode de réalisation particulier, l'au moins une huile est comprise dans le lait d'amande douce qui comprend de l'huile d'amande douce.

Selon un autre mode de réalisation préféré, la présente invention concerne une composition cosmétique telle que définie ci-dessus, dans laquelle ladite composition comprend, en pourcentage en poids par rapport au poids total de la composition :

- un agent d'adhésion des poudres, notamment l'amidon de maïs, à raison de 8 à 11%,
- du mannitol à raison de 7 à 9%,
- de l'$\alpha$-glucan oligosaccharide, à raison de 0,5 à 1,5%,
- du Ghassoul, à raison de 4 à 6%,
- du cocoyl glutamate de sodium à raison de 25 à 35%,
- du cocoyl iséthionate de sodium à raison de 35 à 45%,
- de poudre de bambou à raison de 2 à 4%,
- de l'huile de jojoba à raison de 1 à 3%, et
- du parfum herbes et thé à raison de 1 à 3%,

ladite composition étant notamment un shampoing.

Selon un autre mode de réalisation préféré, la présente invention concerne une composition cosmétique telle que définie ci-dessus, dans laquelle ladite composition comprend, en pourcentage en poids par rapport au poids total de la composition :

- du cocoyl glutamate de sodium à raison de 25 à 35%,
- du cocoyl iséthionate de sodium à raison de 35 à 45%,
- un agent d'adhésion des poudres, notamment l'amidon de maïs,
- de l'$\alpha$-glucan oligosaccharide, à raison de 0,5 à 1,5%,
- de l'huile de tournesol,
- de l'extrait de fleur de camomille,
- de la piroctone olamine
- de l'acide de fleurs, et
- du parfum herbes et thé,

ladite composition étant notamment un shampoing.

Selon un autre mode de réalisation préféré, la présente invention concerne une composition cosmétique telle que définie ci-dessus, dans laquelle ladite composition comprend, en pourcentage en poids par rapport au poids total de la composition :

- du cocoyl glutamate de sodium à raison de 25 à 35%,
- du cocoyl iséthionate de sodium à raison de 35 à 45%,
- un agent d'adhésion des poudres, notamment l'amidon de maïs,
- de l'$\alpha$-glucan oligosaccharide, à raison de 0,5 à 1,5%,
- de l'huile d'argan,
- de la protéine de riz,
- de la protéine de poids,
- de l'acide benzène sulfonique 2-[(9,10-dihydro-4-hydroxy-9,10-dioxo-1-anthracényl) amino]-5-méthyl monosel de sodium,
- de l'erythrol, et
- du parfum herbes et thé,

ladite composition étant notamment un shampoing.

Selon un autre mode de réalisation préféré, la présente invention concerne une composition cosmétique telle que définie ci-dessus, dans laquelle ladite composition comprend, en pourcentage en poids par rapport au poids total de la composition :

- un agent d'adhésion des poudres, notamment l'amidon de maïs, à raison de 75 à 79%,
- du mannitol à raison de 12 à 14%,
- de l'$\alpha$-glucan oligosaccharide, à raison de 0,5 à 1,5%,
- du cocoyl glutamate de sodium à raison de 4 à 6%,

- de l'huile de sésame à raison de 0,5 à 2%,
- de l'huile de jojoba à raison de 0,5 à 2%,
- du zinc PCA à raison de 0,5 à 2%, et
- du charbon à raison de 0,5 à 2%,

ladite composition étant notamment un nettoyant.

Selon un autre mode de réalisation préféré, la présente invention concerne une composition cosmétique telle que définie ci-dessus, dans laquelle ladite composition comprend, en pourcentage en poids par rapport au poids total de la composition :

- un agent d'adhésion des poudres, notamment l'amidon de maïs, à raison de 72 à 76%,
- du mannitol à raison de 9 à 11%,
- de l'$\alpha$-glucan oligosaccharide, à raison de 0,5 à 1,5%,
- du cocoyl glutamate de sodium à raison de 4 à 6%,
- de l'huile d'amande douce à raison de 2 à 4%,
- de l'ascorbyl glucoside à raison de 2 à 4%,
- de l'huile de jojoba à raison de 2 à 4%, et
- de l'acide de fleurs à raison de 1 à 3%,

ladite composition étant notamment un nettoyant.

Selon un autre mode de réalisation préféré, la présente invention concerne une composition cosmétique telle que définie ci-dessus, dans laquelle ladite composition comprend, en pourcentage en poids par rapport au poids total de la composition :

- un agent d'adhésion des poudres, notamment l'amidon de maïs, à raison de 72 à 76%,
- du mannitol à raison de 11 à 13%,
- de l'$\alpha$-glucan oligosaccharide, à raison de 0,5 à 1,5%,
- du cocoyl glutamate de sodium à raison de 4 à 6%,
- de l'huile de camélia à raison de 2 à 4%,
- de l'huile de jojoba à raison de 1 à 3%, et
- du thé matcha à raison de 1 à 3%,

ladite composition étant notamment un nettoyant.

Selon un autre mode de réalisation préféré, la présente invention concerne une composition cosmétique telle que définie ci-dessus, dans laquelle ladite composition comprend, en pourcentage en poids par rapport au poids total de la composition :

- un agent d'adhésion des poudres, notamment l'amidon de maïs, à raison de 65 à 75%,
- du mannitol à raison de 14 à 16%,
- de l'$\alpha$-glucan oligosaccharide, à raison de 0,5 à 1,5%,
- du cocoyl glutamate de sodium à raison de 4 à 6%,
- de l'huile de carthame à raison de 2 à 4%,
- de l'huile de jojoba à raison de 2 à 4%, et
- de la spiruline à raison de 2 à 4%,

ladite composition étant notamment un nettoyant.

Selon un autre mode de réalisation préféré, la présente invention concerne une composition cosmétique telle que définie ci-dessus, dans laquelle ladite composition comprend, en pourcentage en poids par rapport au poids total de la composition :

- un agent d'adhésion des poudres, notamment l'amidon de maïs, à raison de 70 à 76%,
- du mannitol à raison de 13 à 17%,
- de l'$\alpha$-glucan oligosaccharide, à raison de 0,5 à 1,5%,
- du cocoyl glutamate de sodium à raison de 4 à 6%,
- de l'huile de carthame à raison de 1 à 3%,
- de l'huile d'argan à raison de 1 à 3%,
- de l'huile de chanvre à raison de 1 à 3%, et

ladite composition étant notamment un nettoyant.

Selon un autre mode de réalisation particulier, la présente invention concerne une association telle que définie ci-dessus, ou une composition cosmétique telle que définie ci-dessus, ladite association ou composition étant formulée dans une capsule.

La capsule utilisable dans la présente invention est notamment une capsule hydrosoluble, ladite capsule pouvant être solubilisée dans de l'eau lors de l'utilisation, libérant ainsi le contenu de la capsule. De façon alternative, si la capsule n'est pas hydrosoluble, le contenu peut être libéré par ouverture de la capsule. L'homme de l'art pourra choisir une capsule en fonction du besoin parmi les capsules disponibles commercialement, notamment dans le domaine pharmaceutique, ou des compléments alimentaires.

Tout comme les constituants et ingrédients de l'association et ceux de la composition cosmétique de la présente invention, les capsules sont fabriquées à partir de matières naturelles et d'origine non-animale, comme par exemple l'hydroxyméthyl cellulose, les alginates ou l'amidon. La taille des capsules peut être choisie en fonction de la quantité totale de produit à être contenu.

Selon un autre mode de réalisation particulier, la présente invention concerne une association ou une composition cosmétique telle que définie ci-dessus, dans laquelle ladite association ou ladite composition est comprise dans une capsule, à raison de 0,5 à 2.5 grammes, de préférence de 0,75 à 1,25 g, ou de 1,75 à 2,25 g, notamment d'environ 1 grammes, ou 2 grammes.

Lors de l'application de l'association ou de la composition cosmétique, une solution aqueuse éphémère est obtenue, dans laquelle les constituants et les ingrédients sont en solution. Certains ingrédients, comme par exemple le charbon ou le ghassoul peuvent toutefois être en suspension dans ladite solution éphémère.

Un autre objet de la présente invention est une solution aqueuse comprenant une association telle que définie ci-dessus, ou une composition cosmétique telle que définie ci-dessus,

ladite association ou composition étant présente dans une concentration d'environ 10 g/L, ladite solution aqueuse ayant un pH compris de 4 à 6.

Par « *environ 10 g/L* » on entend une valeur comprise de 9 à 11 g/L.

Une solution aqueuse comprenant une association ou d'une composition cosmétique a un pH compatible avec un usage cosmétique sur la peau dans le sens où le pH de la solution est proche du pH de la peau.

Un autre objet de la présente invention est un dispositif comprenant

- une capsule, notamment une capsule hydrosoluble, et
- une association telle que définie ci-dessus, ou une composition cosmétique telle que définie ci-dessus, notamment en quantité comprise de 0,5 à 2.5 grammes, de préférence de 0,75 à 1,25 g, ou de 1,75 à 2,25 g, notamment d'environ 1 grammes, ou 2 grammes.

ladite association ou composition cosmétique étant contenue au sein de la capsule.

Les nettoyants selon la présente invention sont utilisés en quantité comprise de 1 à 2 grammes, notamment d'environ 1 gramme, tandis-que les shampoings sont utilisés en quantité d'environ 1 gramme pour les cheveux courts et d'environ 2 grammes pour les cheveux longs.

Un autre objet de la présente invention est l'utilisation d'une association telle que définie ci-dessus, ou d'une composition cosmétique telle que définie ci-dessus, ou d'un dispositif tel que défini ci-dessus, pour le soin des cheveux ou de la peau.

Un autre objet de la présente invention est une méthode de traitement cosmétique comprenant :

- une étape de solubilisation d'une association telle que définie ci-dessus, ou d'une composition cosmétique telle que définie ci-dessus, ou d'un dispositif tel que défini ci-dessus, par ajout d'eau, pour obtenir une solution aqueuse, et
- une étape d'application sur les cheveux ou sur la peau de ladite solution aqueuse,

la quantité totale d'eau utilisée dans l'étape de solubilisation est notamment compris de 300 à 500% en pourcentage en poids par rapport au poids de l'association, de la composition cosmétique, ou du dispositif.

Un autre objet de la présente invention est une méthode de traitement cosmétique comprenant :

- une étape d'ouverture de la capsule, dans la paume de la main, pour libérer l'association telle que définie ci-dessus, ou la composition cosmétique telle que définie ci-dessus,
- une étape de solubilisation de ladite association, ou de ladite composition cosmétique, par ajout d'eau, pour obtenir une solution aqueuse, et
- une étape d'application sur les cheveux ou sur la peau de ladite solution aqueuse,

la quantité totale d'eau utilisée dans l'étape de solubilisation étant notamment compris de 300 à 500% en pourcentage en poids par rapport au poids de l'association ou de la composition cosmétique.

Les Inventeurs ont trouvé que les shampoings de la présente invention, comprenant notamment deux tensioactifs, ont des propriétés intéressantes, notamment par rapport aux caractéristiques sensorielles tel que le pouvoir moussant, le pouvoir lavant, le pouvoir démêlant, la rinçabilité et la facilité de coiffage.

Les inventeurs ont également trouvé que les nettoyants de la présente invention, comprenant notamment un seul tensioactif, ont des propriétés intéressantes, notamment par rapport aux caractéristiques sensorielles tel que le pouvoir moussant au contact de l'eau, le pouvoir démaquillant, et la rinçabilité.

Les exemples suivants illustrent l'invention, sans en limiter la portée.

## EXEMPLES

**Exemple A** Procédure générale de la fabrication des compositions

Les différents ingrédients sont pesés et ajoutés un à un dans un mixeur. Les poudres sont ajoutées dans un premier temps et l'huile végétale et le parfum sont ajoutés dans un second temps. Les ingrédients sont mélangés pendant 10 secondes à deux reprises et sont ensuite conditionnés.

**Exemple B** Procédure de mesure de densité des poudres

Une éprouvette de 250 ml a été pesée à vide pour donner son poids M0 en gramme. Un volume V de la poudre, compris de 240 à 250 ml, est versé dans l'éprouvette à l'aide d'un entonnoir. L'éprouvette est de nouveau pesée pour donner le poids M1 en gramme.

La densité $\rho$ de la poudre, exprimée en g/ml, est calculée selon la formule 1 suivante :

$$\rho = \frac{M1-M0}{V} \quad \text{formule 1}$$

**Exemple C** Procédure de mesure de pH

La composition est pesée et diluée dans de l'eau pour obtenir une solution aqueuse de la composition à 10% en poids. Le pH est ensuite mesuré à température ambiante, à l'aide d'un pH mètre.

**Exemple D** Procédure générale des tests sensoriels nettoyants sur panel d'experts.

Des tests sensoriels ont été effectués sur un panel d'experts.

Le nettoyant en poudre a été utilisé comme un nettoyant visage classique. Il a été utilisé de la façon suivante :

1) Mouiller le visage
2) Saupoudrer 1 cuillère à café du nettoyant en poudre dans la main
3) Ajouter quelques gouttes d'eau pour le faire mousser entre les mains
4) Appliquer le nettoyant sur le visage et masser pour éliminer le maquillage et les impuretés
5) Rincer à l'eau

La performance du nettoyant est évaluée selon :

- son **pouvoir moussant** au contact de l'eau
- son **pouvoir démaquillant**
- sa **rinçabilité**

**Exemple E** Procédure générale des tests shampoings sur panel d'experts.

Des tests sensoriels ont été effectués sur un panel d'experts.

Le shampoing en poudre a été utilisé sur cheveux mouillés comme un shampoing classique. Il a été utilisé de la façon suivante :

1) Mouiller ses cheveux et ses mains
2) Saupoudrer 1 à 2 cuillères à café du shampoing en poudre dans la main selon la longueur des cheveux et ajouter de l'eau si nécessaire

3) Appliquer le shampoing sur les cheveux
4) Masser les cheveux et le cuir chevelu
5) Rincer à l'eau

La performance du shampoing est évaluée selon :

- son **pouvoir moussant**
- son **pouvoir lavant**
- son **pouvoir démêlant**
- sa **rinçabilité**
- sa **facilité de coiffage**

**Exemple 1** Shampoing au Ghassoul

La composition shampoing du **tableau 1**, comprenant du Ghassoul, a été fabriquée selon la procédure générale de l'exemple A.

**Tableau 1** Composition d'un shampoing au Ghassoul

| nom | nom commercial | nom INCI | % |
|---|---|---|---|
| Amidon de maïs | MAISITA 9060 | Zea Mays (Corn) Starch | 9 |
| Mannitol | Beauté by Roquette® PO260 | Mannitol | 8 |
| Cocoyl glutamate de sodium | AMISOFT® CS 11 | SODIUM COCOYL GLUTAMATE | 30 |
| Cocoyl iséthionate de sodium | PUREACT I-80P | SODIUM COCOYL ISETHIONATE | 40 |
| Ghassoul | GHASSOUL | Morrocan Lava Clay | 5 |
| Poudre de bambou | Greensil | Bambusa Arundinacea Stem Extract | 3 |
| alpha-Glucan oligosaccharide | BIOECOLIA® | $\alpha$-glucan oligosaccharide | 1 |
| Huile de jojoba | Jojoba Oil CP Org | SIMMONDSIA CHINENSIS SEED OIL | 2 |
| Parfum herbes & thé | HERBES & THE 2 NATFLOR RT4693 | | 2 |
| | | TOTAL | 100 |

La composition se présente sous la forme d'une poudre blanche dont la densité, mesurée selon la procédure de l'exemple B, est de 0,42 g/ml.

Le pH, mesuré selon la procédure de l'exemple C, est de 5,85.

**Tests sensoriels**

Le shampoing a été évalué selon la procédure de l'exemple E :

- **pouvoir moussant :** La formation de la mousse est rapide. La mousse est abondante, légère et agréable au toucher.
- **pouvoir lavant :** Les cheveux sont propres après lavage.
- **pouvoir démêlant :** Les cheveux sont faciles à démêler.
- **rinçabilité :** Le shampoing se rince très rapidement.
- **facilité de coiffage :** Les cheveux faciles à démêler au peignage.

**Exemple 2** Shampoing au Ghassoul

La composition shampoing du **tableau 2**, comprenant du Ghassoul, a été fabriquée selon la procédure générale de l'exemple A.

Tableau 2 Composition d'un shampoing au Ghassoul

| nom | Nom INCI | % |
|---|---|---|
| Amidon de maïs | Zea mays (corn) starch | 8 |
| Mannitol | Mannitol | 8 |
| Cocoyl glutamate de sodium | Sodium cocoyl glutamate | 30 |
| Cocoyl iséthionate de sodium | Sodium cocoyl isethionate | 40 |
| Ghassoul | Maroccan lava clay | 5 |
| L-Arginine | L-Arginine | 1 |
| Poudre de bambou | Bambusa arundinacea stem powder | 3 |
| Huile de jojoba | Simmondsia chinensis seed oil | 2 |
| alpha-Glucan oligosaccharide | $\alpha$-glucan oligosaccharide | 1 |
| Parfum herbes & thé | Parfum | 2 |
| | Total | 100 |

La composition se présente sous la forme d'une poudre blanche dont la densité, mesurée selon la procédure de l'exemple B, est de 0,43 g/ml.

Le pH, mesuré selon la procédure de l'exemple C, est de 5,7.

**Tests sensoriels**

Le shampoing a été évalué selon la procédure de l'exemple E :

- **pouvoir moussant :** La formation de la mousse est rapide. La mousse est abondante, légère et agréable au toucher.
- **pouvoir lavant** : Les cheveux sont propres après lavage.
- **pouvoir démêlant** : Les cheveux sont faciles à démêler.
- **rinçabilité** : Le shampoing se rince très rapidement.
- **facilité de coiffage** : Les cheveux faciles à démêler au peignage.

**Exemple 3** Shampoing huile d'argan

La composition shampoing du **tableau 3**, comprenant de l'huile d'argan, a été fabriquée selon la procédure générale de l'exemple A.

Tableau 3 Composition d'un shampoing huile d'argan

| Nom | nom commercial | nom INCI | % |
|---|---|---|---|
| Amidon de maïs | MAISITA 9060 | Zea Mays (Corn) Starch | 9,5 |
| Mannitol | Beauté by Roquette® PO260 | Mannitol | 10 |
| Cocoyl glutamate de sodium | AMISOFT® CS 11 | SODIUM COCOYL GLUTAMATE | 30 |
| Cocoyl iséthionate de sodium | PUREACT I-80P | SODIUM COCOYL ISETHIONATE | 40 |
| Protéine de pois | Promois WJ-SP | Hydrolyzed Pea Protein | 2,5 |
| Aloe vera | ALOE VERA EXTRAIT SEC ATOMISE BIO | Aloe Barbadensis Leaf Juice Powder | 1 |
| $\alpha$-glucan oligosaccharide | BIOECOLIA® | $\alpha$-glucan oligosaccharide | 1 |
| Huile d'argan | Huile d'argan désodorisée | Argania Spinosa Kernel Oil | 4 |

(suite)

| Nom | nom commercial | nom INCI | % |
|---|---|---|---|
| Parfum abricot amande | ABRICOT AMANDE 2 NATFLOR RT4688 | | 2 |
| | | TOTAL | 100 |

La composition se présente sous la forme d'une poudre blanche dont la densité, mesurée selon la procédure de l'exemple B, est de 0,40 g/ml.

Le pH, mesuré selon la procédure de l'exemple C, est de 5,49.

**Exemple 4** Shampoing huile d'argan

La composition shampoing du **tableau 4**, comprenant de l'huile d'argan, a été fabriquée selon la procédure générale de l'exemple A.

**Tableau 4** Composition d'un shampoing huile d'argan

| Nom | nom INCI | % |
|---|---|---|
| Amidon de maïs | Zea Mays (Corn) Starch | 10 |
| Mannitol | Mannitol | 10 |
| Cocoyl glutamate de sodium | SODIUM COCOYL GLUTAMATE | 30 |
| Cocoyl iséthionate de sodium | SODIUM COCOYL ISETHIONATE | 40 |
| Protéine de pois | Hydrolyzed Pea Protein | 2 |
| Aloe vera | Aloe Barbadensis Leaf Juice Powder | 1 |
| $\alpha$-glucan oligosaccharide | $\alpha$-glucan oligosaccharide | 1 |
| Huile d'argan | Argania Spinosa Kernel Oil | 4 |
| Parfum | | 2 |
| | TOTAL | 100 |

La composition se présente sous la forme d'une poudre blanche dont la densité, mesurée selon la procédure de l'exemple B, est de 0,34 g/ml.

Le pH, mesuré selon la procédure de l'exemple C, est de 5,8.

**Tests sensoriels**

Le shampoing a été évalué selon la procédure de l'exemple E :

- **pouvoir moussant** : La formation de la mousse est rapide. La mousse est abondante, légère et agréable au toucher.
- **pouvoir lavant** : Les cheveux sont propres après lavage.
- **pouvoir démêlant** : Les cheveux sont faciles à démêler.
- **rinçabilité** : Le shampoing se rince très rapidement.
- **facilité de coiffage** : Les cheveux faciles à démêler au peignage.

**Exemple 5** Shampoing lait d'amande douce

La composition shampoing du **tableau 5**, comprenant du lait d'amande douce, a été fabriquée selon la procédure générale de l'exemple A.

**Tableau 5** Composition d'un shampoing lait d'amande douce

| nom | nom commercial | nom INCI | % |
|---|---|---|---|
| Amidon de maïs | MAISITA 9060 | Zea Mays (Corn) Starch | 11 |
| Mannitol | Beauté by Roquette PO 260 | Mannitol | 9,5 |
| Cocoyl glutamate de sodium | AMISOFT® CS 11 | SODIUM COCOYL GLUTAMATE | 30 |
| Cocoyl iséthionate de sodium | PUREACT I-80P | SODIUM COCOYL ISETHIONATE | 40 |
| Lait d'amande douce | LAIT AMANDE DOUCE BIO | Prunus Amygdalus Dulcis Protein Oryza Sativa Extract Acacia Senegal Gum Vanilla Planifolia Extract Prunus Amygdalus Dulcis Fruit Extract | 6,5 |
| $\alpha$-glucan oligosaccharide | BIOECOLIA® | $\alpha$-glucan oligosaccharide | 1 |
| Parfum lait de coco | LAIT DE COCO 2 NATFLOR RT4683 | | 2 |
| | | TOTAL | 100 |

La composition se présente sous la forme d'une poudre blanche dont la densité, mesurée selon la procédure de l'exemple B, est de 0,48 g/ml.

Le pH, mesuré selon la procédure de l'exemple C, est de 5,84.

**Exemple 6** Shampoing lait d'amande douce

La composition shampoing du **tableau 6**, comprenant du lait d'amande douce, a été fabriquée selon la procédure générale de l'exemple A.

**Tableau 6** Composition d'un shampoing lait d'amande douce

| nom | nom INCI | % |
|---|---|---|
| Amidon de maïs | Zea mays (corn) starch | 11 |
| Mannitol | Mannitol | 9,5 |
| Cocoyl glutamate de sodium | Sodium cocoyl glutamate | 30 |
| Cocoyl iséthionate de sodium | Sodium cocoyl isethionate | 40 |
| Lait d'amande douce | Lait d'amande douce | 4 |
| Protéines de riz hydrolysées | Hydrolyzed rice bran protein | 2 |
| Extrait de Kappaphycus alvarezii & Extrait de fruit de tara | Kappaphycus alvarezii Extract & Caesalpinia spinosa Fruit Extract | 0,5 |
| $\alpha$-glucan oligosaccharide | $\alpha$-glucan oligosaccharide | 1 |
| Parfum | | 2 |
| | Total | 100 |

La composition se présente sous la forme d'une poudre blanche dont la densité, mesurée selon la procédure de l'exemple B, est de 0,44 g/ml.

Le pH, mesuré selon la procédure de l'exemple C, est de 5,7.

**Tests sensoriels**

Le shampoing a été évalué selon la procédure de l'exemple E :

- **pouvoir moussant** : La formation de la mousse est rapide. La mousse est abondante, légère et agréable au toucher.
- **pouvoir lavant** : Les cheveux sont propres après lavage.
- **pouvoir démêlant** : Les cheveux sont faciles à démêler.
- **rinçabilité** : Le shampoing se rince très rapidement.
- **facilité de coiffage** : Les cheveux faciles à démêler au peignage.

**Exemple 7** Shampoing aux 3 huiles

La composition shampoing du **tableau 7**, comprenant trois huiles, a été fabriquée selon la procédure générale de l'exemple A.

**Tableau 7** Composition d'un shampoing aux trois huiles

| nom | nom commercial | nom INCI | % |
|---|---|---|---|
| Amidon de maïs | MAISITA 9060 | Zea Mays (Corn) Starch | 6 |
| Mannitol | Beauté by Roquette® PO260 | Mannitol | 10 |
| Cocoyl glutamate de sodium | AMISOFT® CS 11 | SODIUM COCOYL GLUTAMATE | 30 |
| Cocoyl iséthionate de sodium | PUREACT I-80P | SODIUM COCOYL ISETHIONATE | 40 |
| Aloe vera | ALOE VERA EXTRAIT SEC ATOMISE BIO | Aloe Barbadensis Leaf Juice Powder | 5 |
| $\alpha$-glucan oligosaccharide | BIOECOLIA® | $\alpha$-glucan oligosaccharide | 1 |
| Huile de graine de moringa | ORGANIC REFINED MORINGA OIL | Moringa Oleifera Seed Oil | 2 |
| Huile de jojoba | Jojoba Oil CP Org | SIMMONDSIA CHINENSIS SEED OIL | 2 |
| Huile d'argan | Huile d'argan désodorisée | Argania Spinosa Kernel Oil | 2 |
| | ABRICOT AMANDE 2 NATFLOR RT4688 | | 2 |
| | | TOTAL | 100 |

La composition se présente sous la forme d'une poudre blanche.
Le pH, mesuré selon la procédure de l'exemple C, est de 5,71.

**Exemple 8** Nettoyant au charbon

La composition nettoyante du **tableau 8**, comprenant du charbon, a été fabriquée selon la procédure générale de l'exemple A.

**Tableau 8** Composition d'un nettoyant au charbon

| nom | nom commercial | nom INCI | % |
|---|---|---|---|
| Amidon de maïs | MAISITA 9060 | Zea Mays (Corn) Starch | 77 |
| Mannitol | Beauté by Roquette® PO260 | Mannitol | 13 |
| Huile de jojoba | Jojoba Oil CP Org | SIMMONDSIA CHINENSIS SEED OIL | 1 |
| Huile de sésame | HUILE VIERGE DE SESAME BIO | Sesamum Indicum Seed Oil | 1 |
| Poudre de Charbon | CHARBON VEGETAL POUDRE | Charcoal Powder | 1 |

(suite)

| nom | nom commercial | nom INCI | % |
|---|---|---|---|
| Sel de zinc de l'acide pyrrolidone carboxylique | ZINCIDONE® | Zinc PCA | 1 |
| α-glucan oligosaccharide | BIOECOLIA® | α-glucan oligosaccharide | 1 |
| Cocoyl glutamate de sodium | AMISOFT® CS 11 | Sodium Cocoyl Glutamate | 5 |
| | | TOTAL | 100 |

La composition se présente sous la forme d'une poudre blanche dont la densité, mesurée selon la procédure de l'exemple B, est de 0,4 g/ml.

Le pH, mesuré selon la procédure de l'exemple C, est de 5,3.

**Exemple 9** Nettoyant au charbon

La composition nettoyante du **tableau 9**, comprenant du charbon, a été fabriquée selon la procédure générale de l'exemple A.

**Tableau 9** Composition d'un nettoyant au charbon

| nom | nom INCI | % |
|---|---|---|
| Poudre de marante | Maranta Arundinacea Root Powder | 77 |
| Mannitol | Mannitol | 13 |
| Huile de jojoba | SIMMONDSIA CHINENSIS SEED OIL | 1 |
| Huile de sésame | Sesamum Indicum Seed Oil | 1 |
| Poudre de Charbon | Charcoal Powder | 1 |
| Sel de zinc de l'acide pyrrolidone carboxylique | Zinc PCA | 1 |
| α-glucan oligosaccharide | α-glucan oligosaccharide | 1 |
| Cocoyl glutamate de sodium | Sodium Cocoyl Glutamate | 5 |
| | TOTAL | 100 |

La composition se présente sous la forme d'une poudre blanche dont la densité, mesurée selon la procédure de l'exemple B, est de 0,39 g/ml.

Le pH, mesuré selon la procédure de l'exemple C, est de 5,7.

**Tests sensoriels**

Le nettoyant a été évalué selon la procédure de l'exemple D :

- **pouvoir moussant** : Au contact de l'eau, le nettoyant mousse légèrement et devient crémeux.
- **pouvoir démaquillant** : Le nettoyant nettoie la peau et retire le maquillage. Néanmoins il nécessite d'être couplé à un démaquillant yeux pour parfaire le démaquillage des yeux lorsque ceux-ci sont maquillés.
- **rinçabilité** : Le nettoyant se rince facilement et laisse une sensation de douceur sur la peau après utilisation.

**Exemple 10** Nettoyant aux acides de fleurs

La composition nettoyante du **tableau 10**, comprenant du charbon, a été fabriquée selon la procédure générale de l'exemple A.

**Tableau 10** Composition d'un nettoyant aux acides de fleurs

| nom | nom commercial | nom INCI | % |
|---|---|---|---|
| Amidon de maïs | MAISITA 9060 | Zea Mays (Corn) Starch | 74 |
| Mannitol | Beauté by Roquette® PO260 | Mannitol | 10 |
| Huile de jojoba | Jojoba Oil CP Org | SIMMONDSIA CHINENSIS SEED OIL | 3 |
| Huile d'amande douce | HUILE D'AMANDE DOUCE VIERGE BIO | Prunus Amygdalus Dulcis (Sweet Almond) Oil | 3 |
| Ascorbyl Glucoside | AA2G | Ascorbyl Glucoside | 2 |
| Acide de fleurs | ACIDE DE FLEURS ROUGES EXTRAIT SEC | Hibiscus Sabdariffa Flower Extract, Maltodextrin | 2 |
| $\alpha$-glucan oligosaccharide | BIOECOLIA® | $\alpha$-glucan oligosaccharide | 1 |
| Cocoyl glutamate de sodium | AMISOFT® CS 11 | Sodium Cocoyl Glutamate | 5 |
| | | TOTAL | 100 |

La composition se présente sous la forme d'une poudre blanche dont la densité, mesurée selon la procédure de l'exemple B, est de 0,41 g/ml.

Le pH, mesuré selon la procédure de l'exemple C, est de 4,82.

**Exemple 11** Nettoyant aux acides de fleurs

La composition nettoyante du **tableau 11**, comprenant du charbon, a été fabriquée selon la procédure générale de l'exemple A.

**Tableau 11** Composition d'un nettoyant aux acides de fleurs

| nom | nom INCI | % |
|---|---|---|
| Poudre de Marante | Maranta Arundinacea Root Powder | 74 |
| Mannitol | Mannitol | 10 |
| Huile de jojoba | SIMMONDSIA CHINENSIS SEED OIL | 3 |
| Huile d'amande douce | Prunus Amygdalus Dulcis (Sweet Almond) Oil | 3 |
| Ascorbyl Glucoside | Ascorbyl Glucoside | 2 |
| Acide de fleurs | Hibiscus Sabdariffa Flower Extract, Maltodextrin | 2 |
| $\alpha$-glucan oligosaccharide | $\alpha$-glucan oligosaccharide | 1 |
| Cocoyl glutamate de sodium | Sodium Cocoyl Glutamate | 5 |
| | TOTAL | 100 |

La composition se présente sous la forme d'une poudre blanche dont la densité, mesurée selon la procédure de l'exemple B, est de 0,40 g/ml.

Le pH, mesuré selon la procédure de l'exemple C, est de 5.

**Tests sensoriels**

Le nettoyant a été évalué selon la procédure de l'exemple D :

- **pouvoir moussant** : Au contact de l'eau, le nettoyant mousse légèrement et devient crémeux.
- **pouvoir démaquillant** : Le nettoyant nettoie la peau et retire le maquillage. Néanmoins il nécessite d'être couplé à un démaquillant yeux pour parfaire le démaquillage des yeux lorsque ceux-ci sont maquillés.

- **rinçabilité** : Le nettoyant se rince facilement et laisse une sensation de douceur sur la peau après utilisation.

**Exemple 12** Nettoyant aux trois huiles

La composition nettoyante du **tableau 12**, comprenant des huiles, a été fabriquée selon la procédure générale de l'exemple A.

Tableau 12 Composition d un nettoyant aux trois huiles

| nom | nom commercial | nom INCI | |
|---|---|---|---|
| Amidon de maïs | MAISITA 9060 | Zea Mays (Corn) Starch | 73 |
| Mannitol | Beauté by Roquette® PO260 | Mannitol | 15 |
| Huile d'argan | Huile d'argan désodorisée | Argania Spinosa Kernel Oil | 2 |
| Huile Vierge de Carthame | Huile Vierge de Carthame Bio | Carthamus Tinctorius Seed Oil | 2 |
| Huile de chanvre | Organic Hemp Oil | Cannabis Sativa Seed Oil | 2 |
| $\alpha$-glucan oligosaccharide | BIOECOLIA® | $\alpha$-glucan oligosaccharide | 1 |
| Cocoyl glutamate de sodium | AMISOFT® CS 11 | Sodium Cocoyl Glutamate | 5 |
| | | TOTAL | 100 |

La composition se présente sous la forme d'une poudre blanche dont la densité, mesurée selon la procédure de l'exemple B, est de 0,5 g/ml.
Le pH, mesuré selon la procédure de l'exemple C, est de 5,5.

**Exemple 13** Nettoyant au thé vert matcha

La composition nettoyante du **tableau 13**, comprenant du thé vert matcha, a été fabriquée selon la procédure générale de l'exemple A.

Tableau 13 Composition d'un nettoyant au thé vert matcha

| nom | nom INCI | % |
|---|---|---|
| Poudre de marante | Maranta Arundinacea Root Powder | 74 |
| Mannitol | Mannitol | 12 |
| Huile de jojoba | SIMMONDSIA CHINENSIS SEED OIL | 3 |
| Huile de camélia | Camellia Oleifera Seed Oil | 3 |
| Thé matcha | Camellia Sinensis Leaf Extract | 2 |
| $\alpha$-glucan oligosaccharide | $\alpha$-glucan oligosaccharide | 1 |
| Cocoyl glutamate de sodium | Sodium Cocoyl Glutamate | 5 |
| | TOTAL | 100 |

La composition se présente sous la forme d'une poudre blanche dont la densité, mesurée selon la procédure de l'exemple B, est de 0,43 g/ml.
Le pH, mesuré selon la procédure de l'exemple C, est de 5,4.

**Tests sensoriels**

Le nettoyant a été évalué selon la procédure de l'exemple D :

- **pouvoir moussant** : Au contact de l'eau, le nettoyant mousse légèrement et devient crémeux.
- **pouvoir démaquillant** : Le nettoyant nettoie la peau et retire le maquillage. Néanmoins il nécessite d'être couplé à un démaquillant yeux pour parfaire le démaquillage des yeux lorsque ceux-ci sont maquillés.

- **rinçabilité** : Le nettoyant se rince facilement et laisse une sensation de douceur sur la peau après utilisation.

**Exemple 14** Nettoyant à la spiruline

La composition nettoyante du **tableau 14**, comprenant de la spiruline, a été fabriquée selon la procédure générale de l'exemple A.

**Tableau 14** Composition d'un nettoyant à la spiruline

| nom | nom commercial | nom INCI | % |
|---|---|---|---|
| Poudre de marante | | Maranta Arundinacea Root Powder | 70 |
| Mannitol | | Mannitol | 15 |
| Huile de jojoba | | SIMMONDSIA CHINENSIS SEED OIL | 3 |
| Huile de Carthame | | Carthamus Tinctorius Seed Oil | 3 |
| Spiruline | | Spirulina Platensis Extract | 3 |
| α-glucan oligosaccharide | BIOECOLIA® | α-glucan oligosaccharide | 1 |
| Cocoyl glutamate de sodium | | Sodium Cocoyl Glutamate | 5 |
| | | TOTAL | 100 |

La composition se présente sous la forme d'une poudre blanche dont la densité, mesurée selon la procédure de l'exemple B, est de 0,4 g/ml.

Le pH, mesuré selon la procédure de l'exemple C, est de 5,7.

**Tests sensoriels**

Le nettoyant a été évalué selon la procédure de l'exemple D :

- **pouvoir moussant** : Au contact de l'eau, le nettoyant mousse légèrement et devient crémeux.
- **pouvoir démaquillant** : Le nettoyant nettoie la peau et retire le maquillage. Néanmoins il nécessite d'être couplé à un démaquillant yeux pour parfaire le démaquillage des yeux lorsque ceux-ci sont maquillés.
- **rinçabilité** : Le nettoyant se rince facilement et laisse une sensation de douceur sur la peau après utilisation.

**Exemple 15** Shampoing antipelliculaire

La composition shampoing du **tableau 15** a été fabriquée selon la procédure générale de l'exemple A.

**Tableau 15** Composition d'un shampoing antipelliculaire

| nom | nom commercial | nom INCI | % |
|---|---|---|---|
| Cocoyl isethionate de sodium | Pureact I-80P | Sodium cocoyl isethionate | 40 |
| Cocoyl glutamate de sodium | Amisoft CS 11 | Sodium Cocoyl Glutamate | 30 |
| Amidon de maïs | Maisita 9060 | Zea Mays (Corn) Starch | 17 |
| Huile de tournesol | HUILE DE TOURNESOL DESODO BIO | HELIANTHUS ANNUS SEED OIL | 4 |
| Extrait de fleur de Camomille | Chamomile Herbasec® | Maltodextrin, Chamomilla Recutita Flower Extract | 2 |
| α-glucan oligosaccharide | Bioecolia® | α-glucan oligosaccharide | 3 |

(suite)

| nom | nom commercial | nom INCI | % |
|---|---|---|---|
| piroctone olamine | Kopirox | Piroctone Olamine | 1 |
| Acide de fleurs | ACIDES DE FLEURS ROUGES EXTRAIT SEC (001233) | HIBISCUS SABDARIFFA FLOWER EXTRACT, MALTODEXTRI N | 1 |
| Parfum herbes & thé | | | 2 |
| | TOTAL | | 100 |

**Exemple 16** Shampoing pour cheveux blonds

La composition shampoing du **tableau 16** a été fabriquée selon la procédure générale de l'exemple A.

**Tableau 16** Composition d'un shampoing pour cheveux blonds

| nom | nom commercial | nom INCI | % |
|---|---|---|---|
| Cocoyl isethionate de sodium | | Sodium cocoyl isethionate | 40 |
| Cocoyl glutamate de sodium | | Sodium cocoyl glutamate | 30 |
| Erythriol | Erylite® | Erythriol | 8 |
| Amidon de maïs | | Zea Mays (Corn) Starch | 10,5 |
| Huile d'argan | | | 5 |
| Protéine de pois | | | 1,5 |
| Protéine de riz | | | 1,5 |
| Parfum herbes & thé | | | 2 |
| $\alpha$-glucan oligosaccharide | Bioecolia® | $\alpha$-glucan oligosaccharide | 1 |
| | Unicert violet K7025-J | CI 60730 | 0,5 |
| | | Total | 100 |

**Example 17** tests d'évaluation de la tolérance clinique de deux shampoings

Les shampoings suivants ont été testés dans un test d'évaluation de la tolérance clinique et dermatologique et de l'acceptabilité d'un produit cosmétique après utilisation sur une période de 21 jours dans les conditions normales d'emploi.

- Le shampoing lait d'amande selon l'exemple 5, sur 21 volontaires
- Le shampoing huile d'argan selon l'exemple 3, sur 22 volontaires

Les tests ont été réalisés par la société IDEA Clinic, France.

• *Mode d'emploi retenu pour l'étude*

Utiliser le shampooing selon votre routine habituelle :

- Bien agiter avant emploi ;
- Appliquer sous la douche, bien mouiller vos cheveux et vos mains ;
- Verser 1/2 cuillère à café de shampooing en poudre dans la main en fonction de la longueur de vos cheveux ;
- Rajouter de l'eau dans vos mains pour faire mousser ;
- Appliquer sur les cheveux et faire mousser comme un shampooing classique ;
- Bien rincer à l'eau.

Les volontaires ont évalué le produit pour ses propriétés après utilisation sur une période de 21 jours. L'étude était monocentrique, ouverte et comparative entre J21 et J1.

**• Résultats - discussion**

Aucun signe clinique d'intolérance n'a été observé pour les deux shampoings.

**Shampoing lait d'amande**

- Items relatifs à l'efficacité du produit (réponses positives ≥ 75 %, seuil de significativité statistique pour 21 volontaires analysés)

  - Le produit est doux pour les cheveux ainsi que pour le cuir chevelu (85,71%)
  - Le produit est adapté à votre type de cheveux (76,19%)
  - Le produit nettoie les cheveux en douceur (90,48%)
  - Le produit protège les cheveux et le cuir chevelu (85,71%)
  - Les cheveux sont légers (76,19%)
  - Les cheveux sont souples (85,71%)
  - Les cheveux sont doux et soyeux (80,95%)
  - Les cheveux sont hydratés (85,71%)
  - Les cheveux sont nourris (85,71%)
  - La mousse est fondante (85,71%)
  - Le produit est facile à rincer (90,48%)
  - Le produit se rince plus facilement qu'un shampoing classique (80,95%)

Dans les conditions de l'étude, le produit a présenté :

- une excellente tolérance dermatologique, d'après le barème adopté, sur le cuir chevelu à tendance grasse et sur les cheveux normaux et fins
- une bonne acceptabilité cosmétique, avec 76% d'opinions favorables.

**Shampoing huile d'argan**

- Items relatifs à l'efficacité du produit (réponses positives ≥ 75 %, seuil de significativité statistique pour 22 volontaires analysés)

  - Le produit est facile à rincer (90,91%)
  - Le produit se rince plus rapidement qu'un shampoing classique (77,27%)
  - Le produit est doux pour les cheveux et pour le cuir chevelu (81,82%)
  - Les cheveux sont légers (77,27%)
  - Ma couleur est préservée (77,27%)
  - Le cuir chevelu gratouille moins (87,50%)

Dans les conditions de l'étude, le produit a présenté :

- une excellente tolérance dermatologique, d'après le barème adopté, sur les cheveux secs et colorés et sur le cuir chevelu ;
- une bonne acceptabilité cosmétique, avec 64 % d'opinions favorables.

**Example 18** tests d'évaluation de la tolérance clinique de trois nettoyants

Les nettoyants suivants ont été testés dans un test d'évaluation de la tolérance clinique et dermatologique et de l'acceptabilité d'un produit cosmétique après utilisation sur une période de 21 jours dans les conditions normales d'emploi.

- Le nettoyant au charbon selon l'exemple 8, sur 23 volontaires
- Le nettoyant acide de fleurs hibiscus selon l'exemple 10, sur 22 volontaires
- Le nettoyant aux trois huiles selon l'exemple 12, sur 23 volontaires

Les tests ont été réalisés par la société IDEA Clinic, France.

• *Mode d'emploi retenu pour l'étude*

Une fois par jour, de préférence le soir :

- agiter le nettoyant avant usage,
- verser 1/2 cuillère à café de produit dans la main,
- ajouter de l'eau et faites mousser entre vos mains,
- appliquer sur le visage en massant par mouvements circulaires pour bien le nettoyer et bien rincer à l'eau.

Les volontaires ont évalué le produit pour ses propriétés après utilisation sur une période de 21 jours. L'étude était monocentrique, ouverte et comparative entre J21 et J1.

**Résultats - discussion**

Aucun signe clinique d'intolérance n'a été observé pour les deux shampoings.

**Nettoyant au Charbon**

- Items relatifs à l'efficacité du produit (réponses positives $\geq$ 75 %, seuil de significativité statistique pour 23 volontaires analysés)

    - Le produit nettoie parfaitement la peau (95,65%)
    - Le produit est agréable à utiliser (100%)
    - Le produit est facile à appliquer (95,65%)
    - Le produit est facile à rincer (100%)
    - La peau est douce (95,65%)
    - La peau est plus nette (95,65%)
    - Le produit élimine les impuretés (95,65%)
    - La peau est propre et saine (95,65%)
    - Le produit améliore l'aspect global et l'état de la peau (95,65%)
    - Le produit diminue la sensation de tiraillements et apporte une sensation de confort (95,65%)
    - La peau est purifiée (95,65%)
    - Les pores paraissent resserrés (95,65%)
    - Le produit diminue les imperfections (86,96%)
    - Le produit est doux pour la peau (95,65%)
    - La peau est moins brillante (95,65%)
    - Le produit limite l'excès de sébum (95,65%)
    - Le produit matifie la peau (95,65%)
    - La peau est protégée des agressions extérieures (95,65%)
    - La peau ne tiraille pas (100%)
    - La peau est parfaitement nettoyée (95,65%)
    - Le produit convient à votre type de peau (95,65%)
    - Votre impression sur le produit est bonne (95,65%)

Dans les conditions de l'étude, le produit a présenté :

- une excellente tolérance dermatologique, d'après le barème adopté, sur la peau grasse du visage ;
- et une très bonne acceptabilité cosmétique, avec 96 % d'opinions favorables.

**Nettoyant acide de fleurs hibiscus**

- Items relatifs à l'efficacité du produit (réponses positives ≥ 75 %, seuil de significativité statistique pour 22 volontaires analysés)

  - Le produit nettoie parfaitement la peau (100%)
  - Le produit est agréable à utiliser (95,45%)
  - Le produit est facile à appliquer (95,45%)
  - Le produit est facile à rincer (100%)
  - La peau est douce (100%)
  - La peau est plus nette (100%)
  - Le produit élimine les impuretés (100%)
  - La peau est propre et saine (100%)
  - Le produit améliore l'aspect global et l'état de la peau (100%)
  - Le produit diminue la sensation de tiraillements et apporte une sensation de confort (100%)
  - La peau ne tiraille pas (100%)
  - Le produit convient à votre type de peau (100%)
  - Le teint est plus lumineux (100%)
  - Les pores paraissent resserrés (100%)
  - La peau est lisse (100%)
  - Le produit est doux pour la peau (100%)
  - La peau est protégée des agressions extérieures (100%)
  - Le teint est uniforme (100%)
  - La peau est parfaitement nettoyée (100%)
  - Votre impression sur le produit est bonne (100%)

Dans les conditions de l'étude, le produit a présenté :

- une excellente tolérance dermatologique, d'après le barème adopté, sur la peau normale du visage ;
- une excellente acceptabilité cosmétique, avec 100 % d'opinions favorables.

**Nettoyant aux trois huiles**

- Items relatifs à l'efficacité du produit (réponses positives ≥ 75 %, seuil de significativité statistique pour 23 volontaires analysés)

  - Le produit nettoie parfaitement la peau (100%)
  - Le produit est agréable à utiliser (100%)
  - Le produit est facile à appliquer (100%)
  - Le produit est facile à rincer (100%)
  - La peau est douce (100%)
  - La peau est plus nette (100%)
  - Le produit élimine les impuretés (100%)
  - La peau est propre et saine (100%)
  - Le produit améliore l'aspect global et l'état de la peau (100%)
  - Le produit diminue la sensation de tiraillements et apporte une sensation de confort (100%)
  - Le produit ne dessèche pas la peau (100%)
  - Le produit convient à votre type de peau (100%)
  - La peau est nourrie (100%)
  - La peau est lisse (100%)
  - La peau ne tiraille pas (100%)
  - Le produit est doux pour la peau (100%)
  - La peau est protégée des agressions extérieures (100%)
  - La peau est parfaitement nettoyée (100%)

- Votre impression sur le produit est bonne 100%)

Dans les conditions de l'étude, le produit a présenté :

- une excellente tolérance dermatologique, d'après le barème adopté, sur la peau normale ou sèche du visage ;

- - et une excellente acceptabilité cosmétique, avec 100 % d'opinions favorables.

**Revendications**

1. Association comprenant les 4 constituants suivants :

   • un agent d'adhésion des poudres,
   • de l'$\alpha$-glucan oligosaccharide,
   • au moins une huile, et
   • au moins un tensioactif,
   ladite association étant sous forme de poudre,
   dans laquelle le pourcentage massique d'eau est compris de 0 à 10% par rapport à la masse totale de l'association,
   ledit agent d'adhésion, ledit $\alpha$-glucan oligosaccharide, et ledit au moins un tensioactif étant sous forme de poudre, ladite au moins une huile étant notamment sous forme de poudre.

2. Association selon la revendication 1, dans laquelle :

   • l'agent d'adhésion des poudres est choisi parmi : l'amidon de maïs, la poudre d'acacia, la poudre de maltodextrine, la poudre d'extrait de pomme, la poudre de tapioca, la poudre de riz, l'extrait de son de riz, la poudre de Marante, et un mélange de ceux-ci ; et/ou
   • l'au moins une huile est choisie parmi : l'huile de jojoba, l'huile d'amande douce, l'huile d'amande amère, l'huile d'argan, l'huile de sésame, l'huile de camélia, l'huile de carthame, l'huile de moringa, l'huile de ricin, l'huile de coco, l'huile d'abricot, l'huile de lin, l'huile de grenade, l'huile de pépins de raisin, les huiles de figues de barbarie, l'huile de marula, l'huile d'onagre, l'huile d'avocat, l'huile de framboise, l'huile de bourrache, l'huile d'olive, l'huile de neem, l'huile de tournesol, l'huile de noix ou de noisette, l'huile d'amla, l'huile de prune, l'huile de café, l'huile de chanvre, un macérat de vanille, une poudre d'huile, une huile fermentée, et un mélange de celles-ci ; et/ou
   • le tensioactif est un tensioactif choisi parmi : lauryl glucoside, decyl glucoside, caprylyl/capryl glucoside, coco-glucoside, hexyl glucoside, sodium cocoyl glutamate, disodium cocoyl glutamate, sodium stearoyl glutamate, sodium lauroyl glutamate, sodium myristoyl glutamate, coco-betaine, cocamidopropyl betaine, disodium cocoamphodiacetate, sodium arganamphoacetate, sodium babassuamphoacetate, sodium cocoabutteramphoacetate, sodium cottonseedamphoacetate, sodium sheabutteramphoacetate, sodium mangoamphoacetate, sodium olivamphoacetate, sodium ricebranamphoacetate, sodium sweetalmondamphoacetate, sodium sunflowerseedamphoacetate, potassium cocoate, potassium palmate, potassium laurate, potassium olivate, sodium coco-sulfate, sodium cocoamphoacetate, disodium coco-glucoside citrate, sodium coco-glucoside tartrate, sodium lauroamphoacetate, cocoyl methyl glucamide, capryloyl/caproyl methyl glucamide, lauroyl/myristoyl methyl glucamide, ammonium lauryl sulfate, ammonium coco-sulfate, magnésium lauryl sulfate, sodium C12-18 alkyl sulfate, zinc coco-sulfate, sodium cocoyl alaninate, sodium lauryl sulfate, sodium cetearyl sulfate, sodium cocoyl isethionate, sodium methyl oleoyl taurate, sodium methyl cocoyl taurate, cocamide MEA, cocamide MIPA, Sodium C14-16 olefin sulfonate, sodium lauryl sulfoacetate, sodium lauroyl sarcosinate, sodium laureth sulfate, ammonium laureth sulfate, cocamidopropyl hydroxysultaine, disodium laureth sulfosuccinate, disodium lauryl sulfosuccinate, sodium myreth sulfate, laureth-5 acide carboxylique, et la saponine,
   ou

   le tensioactif est un tensioactif anionique, notamment choisi parmi : le cocoyl glutamate de sodium, le cocoyl iséthionate de sodium, et un mélange de ceux-ci.

3. Association selon la revendication 1 ou 2, comprenant en outre du mannitol.

4. Association selon la revendication 3, dans laquelle la densité de la poudre est comprise de 0,35 à 0,55 g/ml, notamment de 0,4 à 0,5 g/ml.

5. Association selon la revendication 3 ou 4, dans laquelle :

   • la quantité totale d'agent d'adhésion des poudres est comprise de 5 à 85%, notamment de 5 à 15%, ou de 70 à 85%, en pourcentage en poids par rapport au poids total de l'association ; et/ou
   • la quantité totale de mannitol est comprise de 5 à 20%, notamment de 5 à 15% en pourcentage en poids par rapport au poids total de l'association ; et/ou la quantité totale d'$\alpha$-glucan oligosaccharide est comprise de 0,5 à 2%, notamment d'environ 1% en pourcentage en poids par rapport au poids total de l'association ; et/ou
   • la quantité totale d'huile est comprise de 1 à 50%, notamment de 1 à 10%, notamment de 2 à 8% en pourcentage en poids par rapport au poids total de l'association ; et/ou
   • la quantité totale de tensioactif est comprise de 1 à 80%, notamment de 2 à 20%, ou de 40 à 80% en pourcentage en poids par rapport au poids total de l'association.

6. Association selon l'une quelconque des revendications 3 à 5, ladite association étant sous la forme :

   • d'un shampoing, ledit shampoing comprenant notamment :
   deux tensioactifs en quantité totale comprise de 40 à 80%, notamment de 70 à 80%, en pourcentage en poids par rapport au poids total de la composition, et un agent d'adhésion des poudres, en quantité comprise de 5 à 15% en pourcentage en poids par rapport au poids total de la composition, ou
   • d'un nettoyant, ledit nettoyant comprenant notamment :

      un tensioactif en quantité comprise de 2 à 20%, notamment de 5 à 10% en pourcentage en poids par rapport au poids total de la composition, et
      un agent d'adhésion des poudres, en quantité comprise de 70 à 85% en pourcentage en poids par rapport au poids total de la composition.

7. Utilisation d'une association selon l'une quelconque des revendications 1 à 6, dans une composition cosmétique, notamment pour le soin des cheveux ou de la peau.

8. Composition cosmétique, comprenant une association selon l'une quelconque des revendications 1 à 6, dans un milieu cosmétiquement acceptable,

   ladite composition étant sous la forme d'une poudre,
   dans laquelle le pourcentage massique d'eau est compris de 0 à 10% par rapport à la masse totale de la composition.

9. Composition cosmétique selon la revendication 8, dans laquelle ladite composition comprend en outre un pigment ou de la piroctone olamine.

10. Composition cosmétique selon la revendication 8 ou 9, dans laquelle la densité de la poudre est comprise de 0,35 à 0,55 g/ml, notamment de 0,4 à 0,5 g/ml.

11. Composition cosmétique selon l'une quelconque des revendications 8 à 10, ladite association étant présente à raison de 70 à 100%, notamment de 85 à 100% en pourcentage en poids par rapport au poids total de la composition.

12. Composition cosmétique selon l'une quelconque des revendications 8 à 11, dans laquelle :

   • la quantité totale d'agent d'adhésion des poudres est comprise de 3,5 à 85%, notamment de 3,5 à 15%, ou de 50 à 85%, en pourcentage en poids par rapport au poids total de la composition ; et/ou
   • la quantité totale de mannitol est comprise de 3,5 à 20%, notamment de 8 à 15%, en pourcentage en poids par rapport au poids total de la composition ; et/ou
   • la quantité totale d'$\alpha$-glucan oligosaccharide est comprise de 0.35 à 2%, notamment d'environ 1%, en pourcentage en poids par rapport au poids total de la composition ; et/ou
   • la quantité totale d'huile est comprise de 0,7 à 50% en pourcentage en poids par rapport au poids total de la composition ; et/ou
   • la quantité totale de tensioactif est comprise de 0,7 à 80%, notamment comprise de 1,4 à 20%, ou comprise de 30 à 80% en pourcentage en poids par rapport au poids total de la composition.

**13.** Composition cosmétique selon l'une quelconque des revendications 8 à 12, dans laquelle la composition comprend en outre au moins un ingrédient,

en particulier un principe-actif, notamment choisi parmi :

- l'acide de fleur,
- le Zinc PCA,
- un pigment,
- le charbon,
- un antioxydant, en particulier la vitamine C, ou un de ses dérivés,
- du thé en poudre, notamment du thé vert matcha, du thé fermenté, du thé kombutcha en poudre, la centella asiatica, ou du madécassoside,
- des sucres, notamment le xylitol, le rhamnose, ou les fructooligosaccharides,
- l'aloe vera,
- une protéine, notamment la protéine de pois, la protéine de soja, la protéine de blé, ou la protéine de riz,
- le lait d'amande douce, ou un ou plusieurs de ses composants, notamment le prunus dulcis, le sirop de riz ou la fibre d'acacia,
- un parfum, notamment un parfum abricot amande,
- le ghassoul,
- l'argile blanche, notamment le kaolin
- l'hyaluronate de sodium,
- l'acide hyaluronique,
- l'acide lactique,
- le macérât de vanille,
- la maltodextrine,
- la poudre de bambou,
- la spiruline,
- la phycocianine,
- des champignons fermentés,
- du guarana,
- de la caféine,
- de l'acérola,
- la camomille,
- les poudres ayurvédiques,
- les vitamines, notamment la vitamine B3, ou la vitamine B5,
- les glycosphingolipides et glycolipides,
- les exopolysaccharides,
- les postbiotiques, notamment le lactobacilus ferment,
- la pyroctolamine,
- la piroctone olamine,
- un extrait de graines de camélia,
- un extrait de bleuet,
- un extrait de fleur de camomille, et
- un mélange de ceux-ci,

ledit au moins un ingrédient est notamment solide à température ambiante.

**14.** Composition cosmétique selon l'une quelconque des revendications 8 à 13, ladite composition étant sous la forme :

- d'un shampoing, ledit shampoing comprenant notamment :
deux tensioactifs en quantité totale comprise de 30 à 80%, notamment de 50 à 80% en pourcentage en poids par rapport au poids total de la composition, et un agent d'adhésion des poudres, en quantité comprise de 3,5 à 15% en pourcentage en poids par rapport au poids total de la composition,
ou
- d'un nettoyant, ledit nettoyant comprenant notamment :

un tensioactif à raison de 1,4 à 20% en pourcentage en poids par rapport au poids total de la composition, et un agent d'adhésion des poudres, en quantité comprise de 50 à 85%, notamment de 70 à 85% en pourcentage en poids par rapport au poids total de la composition.

15. Composition cosmétique selon l'une quelconque des revendications 8 à 14, dans laquelle ladite composition comprend :

   • en pourcentage en poids par rapport au poids total de la composition :

   - un agent d'adhésion des poudres, notamment l'amidon de maïs, à raison de 8 à 11%,
   - du mannitol à raison de 9 à 11%,
   - de l'$\alpha$-glucan oligosaccharide, à raison de 0,5 à 1,5%,
   - de l'huile d'Argan, à raison de 3 à 5%,
   - du cocoyl glutamate de sodium à raison de 25 à 35%,
   - du cocoyl iséthionate de sodium à raison de 35 à 45%,
   - de l'aloe vera à raison de 0,5 à 1%,
   - de la protéine de pois à raison de 2 à 3%, et
   - du parfum abricot amande à raison de 1 à 3% ; ou

   • en pourcentage en poids par rapport au poids total de la composition :

   - un agent d'adhésion des poudres, notamment l'amidon de maïs, à raison de 10 à 12%,
   - du mannitol à raison de 9 à 10%,
   - de l'$\alpha$-glucan oligosaccharide, à raison de 0,5 à 1,5%,
   - du lait d'amande douce, à raison de 6 à 7%,
   - du cocoyl glutamate de sodium à raison de 25 à 35%, et
   - du cocoyl iséthionate de sodium à raison de 35 à 45% ; ou

   • en pourcentage en poids par rapport au poids total de la composition :

   - un agent d'adhésion des poudres, notamment l'amidon de maïs, à raison de 8 à 11%,
   - du mannitol à raison de 7 à 9%,
   - de l'$\alpha$-glucan oligosaccharide, à raison de 0,5 à 1,5%,
   - du Ghassoul, à raison de 4 à 6%,
   - du cocoyl glutamate de sodium à raison de 25 à 35%,
   - du cocoyl iséthionate de sodium à raison de 35 à 45%,
   - de poudre de bambou à raison de 2 à 4%,
   - de l'huile de jojoba à raison de 1 à 3%, et
   - du parfum herbes et thé à raison de 1 à 3% ; ou

   • en pourcentage en poids par rapport au poids total de la composition :

   - du cocoyl glutamate de sodium à raison de 25 à 35%,
   - du cocoyl iséthionate de sodium à raison de 35 à 45%,
   - un agent d'adhésion des poudres, notamment l'amidon de maïs,
   - de l'$\alpha$-glucan oligosaccharide, à raison de 0,5 à 1,5%,
   - de l'huile de tournesol,
   - de l'extrait de fleur de camomille,
   - de la piroctone olamine
   - de l'acide de fleurs, et
   - du parfum herbes et thé ; ou

   • en pourcentage en poids par rapport au poids total de la composition :

   - du cocoyl glutamate de sodium à raison de 25 à 35%,
   - du cocoyl iséthionate de sodium à raison de 35 à 45%,
   - un agent d'adhésion des poudres, notamment l'amidon de maïs,
   - de l'$\alpha$-glucan oligosaccharide, à raison de 0,5 à 1,5%,
   - de l'huile d'argan,
   - de la protéine de riz,

- de la protéine de poids,
- de l'acide benzène sulfonique 2-[(9,10-dihydro-4-hydroxy-9,10-dioxo-1-anthracényl) amino]-5-méthyl mono-nosel de sodium,
- de l'erythrol, et
- du parfum herbes et thé ; ou

ladite composition étant notamment un shampoing.

16. Composition cosmétique selon l'une quelconque des revendications 8 à 14, dans laquelle ladite composition comprend :

• en pourcentage en poids par rapport au poids total de la composition :

- un agent d'adhésion des poudres, notamment l'amidon de maïs, à raison de 75 à 79%,
- du mannitol à raison de 12 à 14%,
- de l'α-glucan oligosaccharide, à raison de 0,5 à 1,5%,
- du cocoyl glutamate de sodium à raison de 4 à 6%,
- de l'huile de sésame à raison de 0,5 à 2%,
- de l'huile de jojoba à raison de 0,5 à 2%,
- du zinc PCA à raison de 0,5 à 2%,
- du charbon à raison de 0,5 à 2% ; ou

• en pourcentage en poids par rapport au poids total de la composition :

- un agent d'adhésion des poudres, notamment l'amidon de maïs, à raison de 72 à 76%,
- du mannitol à raison de 9 à 11%,
- de l'α-glucan oligosaccharide, à raison de 0,5 à 1,5%,
- du cocoyl glutamate de sodium à raison de 4 à 6%,
- de l'huile d'amande douce à raison de 2 à 4%,
- de l'ascorbyl glucoside à raison de 2 à 4%,
- de l'huile de jojoba à raison de 2 à 4%, et
- de l'acide de fleurs à raison de 1 à 3% ; ou

• en pourcentage en poids par rapport au poids total de la composition :

- un agent d'adhésion des poudres, notamment l'amidon de maïs, à raison de 72 à 76%,
- du mannitol à raison de 11 à 13%,
- de l'α-glucan oligosaccharide, à raison de 0,5 à 1,5%,
- du cocoyl glutamate de sodium à raison de 4 à 6%,
- de l'huile de camélia à raison de 2 à 4%,
- de l'huile de jojoba à raison de 1 à 3%, et
- du thé matcha à raison de 1 à 3% ; ou

• en pourcentage en poids par rapport au poids total de la composition :

- un agent d'adhésion des poudres, notamment l'amidon de maïs, à raison de 65 à 75%,
- du mannitol à raison de 14 à 16%,
- de l'α-glucan oligosaccharide, à raison de 0,5 à 1,5%,
- du cocoyl glutamate de sodium à raison de 4 à 6%,
- de l'huile de carthame à raison de 2 à 4%,
- de l'huile de jojoba à raison de 2 à 4%, et
- de la spiruline à raison de 2 à 4% ; ou

• en pourcentage en poids par rapport au poids total de la composition :

- un agent d'adhésion des poudres, notamment l'amidon de maïs, à raison de 70 à 76%,
- du mannitol à raison de 13 à 17%,
- de l'α-glucan oligosaccharide, à raison de 0,5 à 1,5%,

- du cocoyl glutamate de sodium à raison de 4 à 6%,
- de l'huile de carthame à raison de 1 à 3%,
- de l'huile d'argan à raison de 1 à 3%, et
- de l'huile de chanvre à raison de 1 à 3%.

ladite composition étant notamment un nettoyant.

**Patentansprüche**

1. Kombination, welche die folgenden 4 Bestandteile umfasst:

   • ein Pulverhaftmittel,
   • $\alpha$-Glucan-Oligosaccharid,
   • mindestens ein Öl, und
   • mindestens ein Tensid,
   wobei die Kombination in Pulverform vorliegt,
   wobei der Massenprozentanteil an Wasser im Bereich von 0 und 10 % liegt, bezogen auf die Gesamtmasse der Kombination,
   wobei das Haftmittel, das $\alpha$-Glucan-Oligosaccharid, und das mindestens ein Tensid in Pulverform vorliegen,
   wobei das mindestens ein Öl insbesondere in Pulverform vorliegt.

2. Kombination nach Anspruch 1, wobei:

   • das Pulverhaftmittel aus Folgendem ausgewählt ist: Maisstärke, Gummi-arabicum-Pulver, Maltodextrin-Pulver, Apfelextraktpulver, Tapiokapulver, Reispulver, Reiskleieextrakt, Pfeilwurzpulver und einer Mischung daraus; und/oder
   • das mindestens eine Öl aus Folgendem ausgewählt ist: Jojobaöl, Süßmandelöl, Bittermandelöl, Arganöl, Sesamöl, Kamelienöl, Distelöl, Behenöl, Rizinusöl, Kokosöl, Aprikosenöl, Leinöl, Granatapfelöl, Traubenkernöl, Kaktusfeigenöle, Amarulaöl, Nachtkerzenöl, Avocadoöl, Himbeersamenöl, Borretschsamenöl, Olivenöl, Niemöl, Sonnenblumenöl, Walnuss- oder Haselnussöl, Amlasamenöl, Pflaumenkernöl, Kaffeeöl, Hanfsamenöl, einem Produkt eingelegter Vanille, einem pulverförmigen Öl, einem fermentierten Öl, einer Mischung daraus; und/oder
   • es sich bei dem Tensid um ein Tensid handelt, das Folgendem ausgewählt ist: Laurylglucosid, Decylglucosid, Caprylyl-/Caprylglucosid, Cocoglucosid, Hexylglucosid, Natriumcocoylglutamat, Dinatriumcocoylglutamat, Natriumstearoylglutamat, Natriumlauroylglutamat, Natriummyristoylglutamat, Cocobetain, Cocamidopropylbetain, Dinatriumcocoamphodiacetat, Natriumarganamphoacetat, Natriumbabassuamphoacetat, Natriumkakaobutteramphoacetat, Natriumbaumwollsaatamphoacetat, Natriumsheabutteramphoacetat, Natriummangoamphoacetat, Natriumolivenamphoacetat, Natriumreiskleieamphoacetat, Natriumsüßmandelamphoacetat, Natriumsonnenblumenkernamphoacetat, Kaliumcocoat, Kaliumpalmat, Kaliumlaurat, Kaliumolivat, Natriumcocosulfat, Natriumcocoamphoacetat, Dinatriumcocoglucosidcitrat, Natriumcocoglucosidtartrat, Natriumlauroamphoacetat, Cocoylmethylglucamid, Caproyl/caproylmethylglucamid, Lauroyl/Myristoylmethylglucamid, Ammoniumlaurylsulfat, Ammoniumcocosulfat, Magnesiumlaurylsulfat, Natrium-C12-18-alkylsulfat, Zinkcocosulfat, Natriumcocoylalaninat, Natriumlaurylsulfat, Natriumcetearylsulfat, Natriumcocoylisethionat, Natriummethyloleoyltaurat, Natriummethylcocoyltaurat, Cocamid MEA, Cocamid MIPA, Natrium-C14-16-olefinsulfonat, Natriumlaurylsulfoacetat, Natriumlauroylsarcosinat, Natriumlaurethsulfat, Ammoniumlaurethsulfat, Cocamidopropylhydroxysultain, Dinatriumlaurethsulfosuccinat, Dinatriumlaurylsulfosuccinat, Natriummyrethsulfat,5-Laurethcarbonsäure und Saponin,

   oder
   es sich bei dem Tensid um ein anionisches Tensid handelt, das insbesondere aus Folgendem ausgewählt ist: Natriumcocoylglutamat, Natriumcocoylisethionat und einer Mischung daraus.

3. Kombination nach Anspruch 1 oder 2, die darüber hinaus Mannit enthält.

4. Kombination nach Anspruch 3, wobei die Dichte des Pulvers 0,35 bis 0,55 g/mL, insbesondere 0,4 bis 0,5 g/mL beträgt.

5. Kombination nach Anspruch 3 oder 4, wobei:

• die Gesamtmenge des Pulverhaftmittels im Bereich von 5 bis 85 %, insbesondere von 5 bis 15 %, oder von 70 bis 85 % liegt, wobei sich der Gewichtsprozentanteil auf das Gesamtgewicht der Kombination bezieht; und/oder

• die Gesamtmenge an Mannit im Bereich von 5 bis 20 %, insbesondere von 5 bis 15 Gew.-% liegt, wobei sich der Gewichtsprozentanteil auf das Gesamtgewicht der Kombination bezieht; und/oder die Gesamtmenge an $\alpha$-Glucan-Oligosaccharid im Bereich von 0,5 bis 2 % liegt, wobei sie insbesondere ungefähr 1 Gew.-% beträgt, wobei sich der Gewichtsprozentanteil auf das Gesamtgewicht der Kombination bezieht; und/oder

• die Gesamtölmenge im Bereich von 1 bis 50 %, insbesondere von 1 bis 10 %, insbesondere von 2 bis 8 Gew.-% liegt, wobei sich der Gewichtsprozentanteil auf das Gesamtgewicht der Kombination bezieht; und/oder

• die Gesamtmenge des Tensids im Bereich von 1 bis 80 %, insbesondere von 2 bis 20 %, oder von 40 bis 80 % liegt, wobei sich der Gewichtsprozentanteil auf das Gesamtgewicht der Kombination bezieht.

6. Kombination nach einem der Ansprüche 3 bis 5, wobei die Kombination in folgender Form vorliegt:

• eines Shampoos, wobei das Shampoo insbesondere Folgendes umfasst:

zwei Tenside in einer Gesamtmenge, die im Bereich von 40 bis 80 %, insbesondere 70 bis 80 % liegt, wobei sich der Gewichtsprozentanteil auf das Gesamtgewicht der Zusammensetzung bezieht, und ein Pulverhaftmittel in einer Menge, die im Bereich von 5 bis 15 % liegt, wobei sich der Gewichtsprozentanteil auf das Gesamtgewicht der Zusammensetzung bezieht, oder

• eines Reinigungsmittels, wobei das Reinigungsmittel insbesondere Folgendes umfasst:

ein Tensid in einer Gesamtmenge, die im Bereich von 2 bis 20 %, insbesondere 5 bis 10 % liegt, wobei sich der Gewichtsprozentanteil auf das Gesamtgewicht der Zusammensetzung bezieht, und ein Pulverhaftmittel in einer Menge, die im Bereich von 70 bis 85 % liegt, wobei sich der Gewichtsprozentanteil auf das Gesamtgewicht der Zusammensetzung bezieht.

7. Verwendung einer Kombination nach einem beliebigen der Ansprüche 1 bis 6 in einer kosmetischen Zusammensetzung, insbesondere zur Pflege der Haare oder der Haut.

8. Kosmetische Zusammensetzung, die eine Kombination nach einem beliebigen der Ansprüche 1 bis 6 in einem kosmetisch unbedenklichen Medium umfasst, wobei die Zusammensetzung in Form eines Pulvers vorliegt, wobei der Massenprozentanteil an Wasser im Bereich von 0 und 10 % liegt, bezogen auf die Gesamtmasse der Zusammensetzung.

9. Kosmetische Zusammensetzung nach Anspruch 8, wobei die Zusammensetzung darüber hinaus ein Pigment oder Pirocton-Olamin umfasst.

10. Kosmetische Zusammensetzung nach Anspruch 8 oder 9, wobei die Dichte des Pulvers im Bereich von 0,35 bis 0,55 g/mL, insbesondere von 0,4 bis 0,5 g/mL liegt.

11. Kosmetische Zusammensetzung nach einem beliebigen der Ansprüche 8 bis 10, wobei die Kombination zu einem Anteil von 70 bis 100 Gew.-%, insbesondere von 85 bis 100 Gew.-% vorliegt, wobei sich der Gewichtsprozentanteil auf das Gesamtgewicht der Zusammensetzung bezieht.

12. Kosmetische Zusammensetzung nach einem beliebigen der Ansprüche 8 bis 11, wobei:

• die Gesamtmenge des Pulverhaftmittels im Bereich von 3,5 bis 85 %, insbesondere von 3,5 bis 15 %, oder von 50 bis 85 % liegt, wobei sich der Gewichtsprozentanteil auf das Gesamtgewicht der Zusammensetzung bezieht; und/oder

• die Gesamtmenge an Mannit im Bereich von 3,5 bis 20 %, insbesondere von 8 bis 15 % liegt, wobei sich der Gewichtsprozentanteil auf das Gesamtgewicht der Zusammensetzung bezieht; und/oder

• die Gesamtmenge an $\alpha$-Glucan-Oligosaccharid im Bereich von 0,35 bis 2 %, wobei sie insbesondere ungefähr 1 % beträgt, wobei sich der Gewichtsprozentanteil auf das Gesamtgewicht der Kombination bezieht; und/oder

• die Gesamtölmenge im Bereich insbesondere von 0,7 bis 50 Gew.-% liegt, wobei sich der Gewichtsprozentanteil auf das Gesamtgewicht der Zusammensetzung bezieht; und/oder

• die Gesamtmenge an Tensid im Bereich von 0,7 bis 80 %, insbesondere im Bereich von 1,4 bis 20 %, oder im Bereich von 30 bis 80 % liegt, wobei sich der Gewichtsprozentanteil auf das Gesamtgewicht der Zusammensetzung bezieht.

13. Kosmetische Zusammensetzung nach einem beliebigen der Ansprüche 8 bis 12, wobei die Zusammensetzung darüber hinaus mindestens einen Inhaltsstoff umfasst,

wobei es sich insbesondere um einen Wirkstoff handelt, der insbesondere aus den folgenden ausgewählt ist:

- Blütensäure,
- Zink-PCA,
- einem Pigment,
- Kohle,
- einem Antioxidans, insbesondere Vitamin C, oder einem der Derivate davon,
- Teepulver, einschließlich grünem Matcha-Tee, fermentiertem Tee, Kombuchatee-Pulver, Centella asiatica oder Madecassosid,
- Zuckerarten, insbesondere Xylit, Rhamnose oder Fructooligosacchariden,
- Aloe vera,
- einem Protein, insbesondere Erbsenprotein, Sojaprotein, Weizenprotein oder Reisprotein,
- Süßmandelmilch oder einem oder mehreren ihrer Bestandteile, insbesondere Prunus dulcis, Reissirup oder Gummi-arabicum-Faser,
- einem Duftstoff, einschließlich eines Aprikosenmandel-Duftstoffs,
- Lavaerde,
- weißer Tonerde, insbesondere Kaolin
- Natriumhyaluronat,
- Hyaluronsäure,
- Milchsäure,
- einem Produkt eingelegter Vanille,
- Maltodextrin,
- Bambuspulver,
- Spirulina,
- Phycocyanin,
- fermentierten Pilzen,
- Guarana,
- Koffein,
- Acerola,
- Kamille,
- ayurvedischen Pulvern,
- Vitaminen wie Vitamin B3 oder Vitamin B5,
- Glycosphingolipiden und Glycolipiden,
- Exopolysacchariden,
- Postbiotika, insbesondere Lactobacillus-Enzym,
- Pyroctolamin,
- Pirocton-Olamin,
- einem Kameliensamen-Extrakt,
- einem Kornblumenextrakt,
- ein Kamillenblüten-Extrakt, und
- einer Mischung davon,

wobei der mindestens eine Inhaltsstoff bei Umgebungstemperatur insbesondere feststofflich vorliegt.

14. Kosmetische Zusammensetzung nach einem beliebigen der Ansprüche 8 bis 13, wobei die Zusammensetzung in folgender Form vorliegt:

- eines Shampoos, wobei das Shampoo insbesondere Folgendes umfasst:

zwei Tenside in einer Gesamtmenge, die im Bereich von 30 bis 80 %, insbesondere 50 bis 80 % liegt, wobei sich der Gewichtsprozentanteil auf das Gesamtgewicht der Zusammensetzung bezieht, und
ein Pulverhaftmittel in einer Menge, die im Bereich von 3,5 bis 15 % liegt, wobei sich der Gewichtsprozentanteil auf das Gesamtgewicht der Zusammensetzung bezieht, oder

• eines Reinigungsmittel, wobei dieses Reinigungsmittel insbesondere Folgendes umfasst:

ein Tensid zu einem Anteil von 1,4 bis 20 %, wobei sich der Gewichtsprozentanteil auf das Gesamtgewicht der Zusammensetzung bezieht, und
ein Pulverhaftmittel in einer Menge, die im Bereich von 50 bis 85 %, insbesondere von 70 bis 85 % liegt, wobei sich der Gewichtsprozentanteil auf das Gesamtgewicht der Zusammensetzung bezieht.

**15.** Kosmetische Zusammensetzung nach einem beliebigen der Ansprüche 8 bis 14, wobei die Zusammensetzung Folgendes umfasst:

• nach Gewichtsprozentanteil unter Bezugnahme auf das Gesamtgewicht der Zusammensetzung:

- ein Pulverhaftmittel, insbesondere Maisstärke, zu einem Anteil von 8 bis 11 %,
- Mannit zu einem Anteil von 9 bis 11 %,
- $\alpha$-Glucan-Oligosaccharid zu einem Anteil von 0,5 bis 1,5 %,
- Arganöl zu einem Anteil von 3 bis 5 %,
- Natriumcocoylglutamat zu einem Anteil von 25 bis 35 %,
- Natriumcocoylisethionat zu einem Anteil von 35 bis 45 %,
- Aleo vera zu einem Anteil von 0,5 bis 1 %,
- Erbsenprotein zu einem Anteil von 2 bis 3 %, und
- Aprikosenmandel-Duftstoff zu einem Anteil von 1 bis 3 %; oder

• nach Gewichtsprozentanteil unter Bezugnahme auf das Gesamtgewicht der Zusammensetzung:

- ein Pulverhaftmittel, insbesondere Maisstärke, zu einem Anteil von 10 bis 12 %,
- Mannit zu einem Anteil von 9 bis 10 %,
- $\alpha$-Glucan-Oligosaccharid zu einem Anteil von 0,5 bis 1,5 %,
- Süßmandelmilch zu einem Anteil von 6 bis 7 %,
- Natriumcocoylglutamat zu einem Anteil von 25 bis 35 %, und
- Natriumcocoylisethionat zu einem Anteil von 35 bis 45 %; oder

• nach Gewichtsprozentanteil unter Bezugnahme auf das Gesamtgewicht der Zusammensetzung:

- ein Pulverhaftmittel, insbesondere Maisstärke, zu einem Anteil von 8 bis 11 %,
- Mannit zu einem Anteil von 7 bis 9 %,
- $\alpha$-Glucan-Oligosaccharid zu einem Anteil von 0,5 bis 1,5 %,
- Lavaerde, zu einem Anteil von 4 bis 6 %,
- Natriumcocoylglutamat zu einem Anteil von 25 bis 35 %,
- Natriumcocoylisethionat zu einem Anteil von 35 bis 45 %,
- Bambuspulver zu einem Anteil von 2 bis 4 %,
- Jojobaöl zu einem Anteil von 1 bis 3 %, und
- Wildkräuter- und Tee-Duftstoff zu einem Anteil von 1 bis 3 %; oder

• nach Gewichtsprozentanteil unter Bezugnahme auf das Gesamtgewicht der Zusammensetzung:

- Natriumcocoylglutamat zu einem Anteil von 25 bis 35 %,
- Natriumcocoylisethionat zu einem Anteil von 35 bis 45 %,
- ein Pulverhaftmittel, insbesondere Maisstärke,
- $\alpha$-Glucan-Oligosaccharid zu einem Anteil von 0,5 bis 1,5 %,
- Sonnenblumenöl,
- Kamillenblüten-Extrakt,
- Pirocton-Olamin
- Blütensäure, und
- Wildkräuter- und Tee-Duftstoff; oder

• nach Gewichtsprozentanteil unter Bezugnahme auf das Gesamtgewicht der Zusammensetzung:

- Natriumcocoylglutamat zu einem Anteil von 25 bis 35 %,
- Natriumcocoylisethionat zu einem Anteil von 35 bis 45 %,
- ein Pulverhaftmittel, insbesondere Maisstärke,
- α-Glucan-Oligosaccharid zu einem Anteil von 0,5 bis 1,5 %,
- Arganöl,
- Reisprotein,
- Erbsenprotein,
- 2-[(9,10-Dihydro-4-hydroxy-9,10-dioxo-1-anthracenyl)amino]-5-methylbenzolsulfonsäure-Mononatriumsalz,
- Erythrol, und
- Wildkräuter- und Tee-Duftstoff; oder

wobei es sich bei dieser Zusammensetzung insbesondere um ein Shampoo handelt.

16. Kosmetische Zusammensetzung nach einem beliebigen der Ansprüche 8 bis 14, wobei die Zusammensetzung Folgendes umfasst:

• nach Gewichtsprozentanteil unter Bezugnahme auf das Gesamtgewicht der Zusammensetzung:

- ein Pulverhaftmittel, insbesondere Maisstärke, zu einem Anteil von 75 bis 79 %,
- Mannit zu einem Anteil von 12 bis 14 %,
- α-Glucan-Oligosaccharid zu einem Anteil von 0,5 bis 1,5 %,
- Natriumcocoylglutamat zu einem Anteil von 4 bis 6 %,
- Sesamöl zu einem Anteil von 0,5 bis 2 %,
- Jojobaöl zu einem Anteil von 0,5 bis 2 %,
- Zink-PCA zu einem Anteil von 0,5 bis 2 %,
- Kohle zu einem Anteil von 0,5 bis 2 %; oder

• nach Gewichtsprozentanteil unter Bezugnahme auf das Gesamtgewicht der Zusammensetzung:

- ein Pulverhaftmittel, insbesondere Maisstärke, zu einem Anteil von 72 bis 76 %,
- Mannit zu einem Anteil von 9 bis 11 %,
- α-Glucan-Oligosaccharid zu einem Anteil von 0,5 bis 1,5 %,
- Natriumcocoylglutamat zu einem Anteil von 4 bis 6 %,
- Süßmandelmilch zu einem Anteil von 2 bis 4 %,
- Ascorbylglucosid zu einem Anteil von 2 bis 4 %,
- Jojobaöl zu einem Anteil von 2 bis 4 %, und
- Blütensäure zu einem Anteil von 1 bis 3 %; oder

• nach Gewichtsprozentanteil unter Bezugnahme auf das Gesamtgewicht der Zusammensetzung:

- ein Pulverhaftmittel, insbesondere Maisstärke, zu einem Anteil von 72 bis 76 %,
- Mannit zu einem Anteil von 11 bis 13 %,
- α-Glucan-Oligosaccharid zu einem Anteil von 0,5 bis 1,5 %,
- Natriumcocoylglutamat zu einem Anteil von 4 bis 6 %,
- Kamelienöl zu einem Anteil von 2 bis 4 %,
- Jojobaöl zu einem Anteil von 1 bis 3 %, und
- Matcha-Tee zu einem Anteil von 1 bis 3 %; oder

• nach Gewichtsprozentanteil unter Bezugnahme auf das Gesamtgewicht der Zusammensetzung:

- ein Pulverhaftmittel, insbesondere Maisstärke, zu einem Anteil von 65 bis 75 %,
- Mannit zu einem Anteil von 14 bis 16 %,
- α-Glucan-Oligosaccharid zu einem Anteil von 0,5 bis 1,5 %,
- Natriumcocoylglutamat zu einem Anteil von 4 bis 6 %,
- Distelöl zu einem Anteil von 2 bis 4 %,
- Jojobaöl zu einem Anteil von 2 bis 4 %, und
- Spirulina zu einem Anteil von 2 bis 4 %; oder

• nach Gewichtsprozentanteil unter Bezugnahme auf das Gesamtgewicht der Zusammensetzung:

- ein Pulverhaftmittel, insbesondere Maisstärke, zu einem Anteil von 70 bis 76 %,
- Mannit zu einem Anteil von 13 bis 17 %,
- α-Glucan-Oligosaccharid zu einem Anteil von 0,5 bis 1,5 %,
- Natriumcocoylglutamat zu einem Anteil von 4 bis 6 %,
- Distelöl zu einem Anteil von 1 bis 3 %,
- Arganöl zu einem Anteil von 1 bis 3 %, und
- Hanfsamenöl zu einem Anteil von 1 bis 3 %, wobei es sich der Zusammensetzung insbesondere um ein Reinigungsmittel.

**Claims**

1. Combination comprising the following 4 constituents:

   • a powder adhesion agent,
   • α-glucan oligosaccharide,
   • at least one oil, and
   • at least one surfactant,
   said combination being in powder form,
   in which the mass percentage of water is comprised from 0 to 10% relative to the total mass of the combination, said adhesion agent, said α-glucan oligosaccharide, and said at least one surfactant being in powder form, said at least one oil being in particular in powder form.

2. Combination according to claim 1, wherein:

   • the powder adhesion agent is chosen from: corn starch, acacia powder, maltodextrin powder, apple extract powder, tapioca powder, rice powder, rice bran extract, and arrowroot powder or a mixture of these; and/or
   • the at least one oil is chosen from: jojoba oil, sweet almond oil, bitter almond oil, argan oil, sesame oil, camellia oil, safflower oil, moringa oil, Castor oil, coconut oil, apricot oil, linseed oil, pomegranate oil, grapeseed oil, prickly pear oils, marula oil, evening primrose oil, avocado oil, raspberry oil, borage oil, olive oil, neem oil, sunflower oil, walnut or hazelnut oil, amla oil, plum oil, coffee oil, hemp oil, a vanilla macerate, an oil powder, and a fermented oil,or a mixture of these; and/or
   • the surfactant is a surfactant chosen from: lauryl glucoside, decyl glucoside, caprylyl/capryl glucoside, coco-glucoside, hexyl glucoside, sodium cocoyl glutamate, disodium cocoyl glutamate, sodium stearoyl glutamate, sodium lauroyl glutamate, sodium myristoyl glutamate, coco-betaine, cocamidopropyl betaine, disodium cocoamphodiacetate, sodium arganamphoacetate , sodium babassuamphoacetate, sodium cocoabutteramphoacetate, sodium cottonseedamphoacetate, sodium sheabutteramphoacetate, sodium mangoamphoacetate, sodium olivamphoacetate, sodium ricebranamphoacetate, sodium sweetalmondamphoacetate, sodium sunflowerseedamphoacetate, potassium cocoate, potassium palmate, potassium laurate, potassium olivate, sodium coco-sulfate, sodium cocoamphoacetate, disodium coco -glucoside citrate, sodium coco-glucoside tartrate, sodium lauroamphoacetate, cocoyl methyl glucamide, capryloyl/caproyl methyl glucamide, lauroyl/myristoyl methyl glucamide, ammonium lauryl sulfate, ammonium coco-sulfate, magnesium lauryl sulfate, sodium C12-18 alkyl sulfate , zinc coco-sulfate, sodium cocoyl alaninate, sodium lauryl sulfate, sodium cetearyl sulfate, sodium cocoyl isethionate, sodium methyl oleoyl taurate, sodium methyl cocoyl taurate, cocamide MEA, cocamide MIPA, sodium C14-16 olefin sulfonate, sodium lauryl sulfoacetate, sodium lauroyl sarcosinate, sodium laureth sulfate, ammonium laureth sulfate, cocamidopropyl hydroxysultaine, disodium laureth sulfosuccinate, disodium lauryl sulfosuccinate, sodium myreth sulfate, laureth-5 carboxylic acid, and saponin,

   or

   the surfactant is an anionic surfactant chosen in particular from: sodium cocoyl glutamate, and sodium cocoyl isethionate, or a mixture of these.

3. Combination according to claim 1 or 2, further comprising mannitol.

4. Combination according to claim 3, wherein the density of the powder is comprised from 0.35 to 0.55 g/ml, in particular from 0.4 to 0.5 g/ml.

**5.** Combination according to claim 3 or 4, wherein:

- the total quantity of powder adhesion agent is comprised from 5 to 85%, in particular from 5 to 15%, or from 70 to 85%, in percentage by weight relative to the total weight of the combination; and/or
- the total quantity of mannitol is comprised from 5 to 20%, in particular from 5 to 15% in percentage by weight relative to the total weight of the combination; and/or
- the total quantity of $\alpha$-glucan oligosaccharide is comprised from 0.5 and 2%, in particular approximately 1% in percentage by weight relative to the weight total of the combination; and/or
- the total quantity of oil is comprised from 1 to 50%, in particular from 1 to 10%, in particular from 2 to 8% in percentage by weight with respect to the total weight of the combination; and/or
- the total quantity of surfactant is comprised from 1 and 80%, in particular from 2 to 20%, or from 40 to 80% in percentage by weight relative to the total weight of the combination.

**6.** Combination according to any of claims 3 to 5, said combination being in the form:

- of a shampoo, said shampoo comprising in particular:
two surfactants in a total amount comprised from 40 to 80%, in particular from 70 to 80%, as a percentage by weight relative to the total weight of the composition, and a powder adhesion agent, in an amount comprised from 5 to 15% in percentage by weight relative to the total weight of the composition, or
- a cleanser, said cleanser comprising in particular:
a surfactant in an amount comprised from 2 to 20%, in particular from 5 to 10% by weight percentage relative to the total weight of the composition, and a powder adhesion agent, in an amount of 70 to 85% in percentage by weight relative to the total weight of the composition.

**7.** Use of a combination according to any of claims 1 to 6, in a cosmetic composition.

**8.** Cosmetic composition, comprising a combination according to any of claims 1 to 6, in a cosmetically acceptable medium,

said composition being in the form of a powder,
in which the mass percentage of water is comprised from 0 to 10% relative to the total mass of the composition.

**9.** Cosmetic composition according to claim 8, wherein said composition further comprises a pigment or piroctone olamine.

**10.** Cosmetic composition according to claim 8 or 9, wherein the density of the powder is comprised from 0.35 to 0.55 g/ml, in particular from 0.4 to 0.5 g/ml.

**11.** Cosmetic composition according to any of claims 8 to 10, said combination being present in an amount of 70 to 100%, in particular 85 to 100% in percentage by weight relative to the total weight of the composition.

**12.** Cosmetic composition according to any of claims 8 to 11, wherein:

- the total quantity of powder adhesion agent is comprised from 3.5 to 85%, in particular from 3.5 to 15%, or from 50 to 85%, as a percentage by weight relative to the total weight of the composition; and/or
- the total quantity of mannitol is comprised from 3.5 to 20%, in particular from 8 to 15%, as a percentage by weight relative to the total weight of the composition; and/or
- the total quantity of $\alpha$-glucan oligosaccharide is comprised from 0.35 to 2%, in particular approximately 1%, as a percentage by weight relative to the total weight of the composition; and/or
- the total quantity of oil is comprised from 0.7 to 50% in percentage by weight relative to the total weight of the composition; and/or
- the total amount of surfactant is comprised from 0.7 to 80%, in particular comprised from 1.4 to 20%, or comprised from 30 to 80% as a percentage by weight relative to the total weight of the composition.

**13.** Cosmetic composition according to any of claims 8 to 12, wherein the composition also comprises at least one ingredient,

in particular an active principle, in particular chosen from:

- flower acid,
- Zinc PCA,
- a pigment,
- the coal,
- an antioxidant, in particular vitamin C, or one of its derivatives,
- powdered tea, including matcha green tea, fermented tea, powdered kombucha tea, centella asiatica, or madecassoside,
- sugars, in particular xylitol, rhamnose, or fructooligosaccharides,
- the aloe vera,
- a protein, in particular pea protein, soy protein, wheat protein, or rice protein,
- sweet almond milk, or one or more of its components, in particular prunus dulcis, rice syrup or acacia fibre,
- a perfume, in particular an apricot almond perfume,
- ghassoul,
- white clay, especially kaolin,
- sodium hyaluronate,
- hyaluronic acid,
- lactic acid,
- vanilla macerate,
- maltodextrin,
- bamboo powder,
- spirulina,
- phycocyanin,
- fermented mushrooms,
- guarana,
- caffeine,
- acerola,
- chamomile,
- ayurvedic powders,
- vitamins, in particular vitamin B3, or vitamin B5,
- glycosphingolipids and glycolipids,
- exopolysaccharides,
- postbiotics, in particular lactobacilus ferment,
- pyroctolamine, piroctone olamine,
- an extract of camellia seeds,
- a corn flower extract,
- a chamomile flower extract, or
- a mixture of these,

said at least one ingredient is in particular solid at room temperature.

14. Cosmetic composition according to any of claims 8 to 13, said composition being in the form:

- of a shampoo, said shampoo comprising in particular:
two surfactants in a total amount comprised from 30 to 80%, in particular from 50 to 80% as a percentage by weight relative to the total weight of the composition, and a powder adhesion agent, in an amount comprised from 3.5 to 15% in percentage by weight relative to the total weight of the composition,
or
- of a cleanser, said cleanser comprising in particular:
a surfactant in an amount of 1.4 to 20% in percentage by weight relative to the total weight of the composition, and a powder adhesion agent, in an amount comprised from 50 to 85%, in particular from 70 to 85% in percentage by weight relative to the total weight of the composition.

15. Cosmetic composition according to any of claims 8 to 14, in which said composition comprises:

- in percentage by weight relative to the total weight of the composition:

  - a powder adhesion agent, in particular corn starch, in an amount of 8 to 11%, and
  - mannitol in an amount of 9 to 11%,

- α-glucan oligosaccharide, in an amount of 0.5 to 1.5%,
- Argan oil, in an amount of 3 to 5%,
- sodium cocoyl glutamate in an amount of 25 to 35%,
- sodium cocoyl isethionate in an amount of 35 to 45%,
- aloe vera in an amount of 0.5 to 1%,
- pea protein at 2 to 3%, and
- apricot almond perfume in an amount of 1 to 3%; or

• in percentage by weight relative to the total weight of the composition:

- a powder adhesion agent, in particular corn starch, in an amount of 10 to 12%,
- mannitol in an amount of 9 to 10%,
- α-glucan oligosaccharide, in an amount of 0.5 to 1.5%,
- sweet almond milk, in an amount of 6 to 7%,
- sodium cocoyl glutamate in an amount of 25 to 35%, and
- sodium cocoyl isethionate in an amount of 35 to 45%; or

• in percentage by weight relative to the total weight of the composition:

- a powder adhesion agent, in particular corn starch, in an amount of 8 to 11%,
- mannitol in an amount of 7 to 9%,
- α-glucan oligosaccharide, in an amount of 0.5 to 1.5%,
- ghassoul, in an amount of 4 to 6%,
- sodium cocoyl glutamate in an amount of 25 to 35%,
- sodium cocoyl isethionate in an amount of 35 to 45%,
- bamboo powder in an amount of 2 to 4%,
- jojoba oil in an amount of 1 to 3%, and
- herbal and tea perfume in an amount of 1 to 3%; or

• in percentage by weight relative to the total weight of the composition:

- sodium cocoyl glutamate in an amount of 25 to 35%,
- sodium cocoyl isethionate in an amount of 35 to 45%,
- a powder adhesion agent, in particular corn starch,
- α-glucan oligosaccharide, in an amount of 0.5 to 1.5%,
- sunflower oil,
- chamomile flower extract,
- piroctone olamine
- flower acid, and
- herbal and tea fragrance; or

• in percentage by weight relative to the total weight of the composition:

- sodium cocoyl glutamate in an amount of 25 to 35%,
- sodium cocoyl isethionate in an amount of 35 to 45%,
- a powder adhesion agent, in particular corn starch,
- α-glucan oligosaccharide, in an amount of 0.5 to 1.5%,
- argan oil,
- rice protein,
- pea protein,
- benzene sulfonic acid 2-[(9,10-dihydro-4-hydroxy-9,10-dioxo-1-anthracenyl) amino]-5-methyl sodium monosalt,
- erythrol, and
- herbal and tea fragrance;

said composition being in particular a shampoo.

16. Cosmetic composition according to any of claims 8 to 14, in which said composition comprises:

• in percentage by weight relative to the total weight of the composition:

- a powder adhesion agent, in particular corn starch, in an amount of 75 to 79%,
- mannitol in an amount of 12 to 14%,
- α-glucan oligosaccharide, in an amount of 0.5 to 1.5%,
- sodium cocoyl glutamate in an amount of 4 to 6%,
- sesame oil in an amount of 0.5 to 2%,
- jojoba oil in an amount of 0.5 to 2%,
- zinc PCA in an amount of 0.5 to 2%, and
- coal in an amount of 0.5 to 2%; or

• in percentage by weight relative to the total weight of the composition:

- a powder adhesion agent, in particular corn starch, in an amount of 72 to 76%,
- mannitol in an amount of 9 to 11%,
- α-glucan oligosaccharide, in an amount of 0.5 to 1.5%,
- sodium cocoyl glutamate in an amount of 4 to 6%,
- sweet almond oil in an amount of 2 to 4%,
- ascorbyl glucoside in an amount of 2 to 4%,
- jojoba oil in an amount of 2 to 4%, and
- flower acid in an amount of 1 to 3%; or

• in percentage by weight relative to the total weight of the composition:

- a powder adhesion agent, in particular corn starch, in an amount of 70 to 76%,
- mannitol in an amount of 11 to 13%,
- α-glucan oligosaccharide, in an amount of 0.5 to 1.5%,
- sodium cocoyl glutamate in an amount of 4 to 6%,
- camellia oil a rate of 2 to 4%,
- jojoba oil in an amount of 2 to 4%, and
- matcha tea in an amount of 1 to 3%; or

• in percentage by weight relative to the total weight of the composition:

- a powder adhesion agent, in particular corn starch, in an amount of 65 to 75%,
- mannitol in an amount of 14 to 16%,
- α-glucan oligosaccharide, in an amount of 0.5 to 1.5%,
- sodium cocoyl glutamate in an amount of 4 to 6%,
- safflower oil in an amount of 2 to 4%,
- jojoba oil in an amount of 2 to 4%, and
- spirulina in an amount of 2 to 4%; or

• in percentage by weight relative to the total weight of the composition:

- a powder adhesion agent, in particular corn starch, in an amount of 70 to 76%,
- mannitol in an amount of 13 to 17%,
- α-glucan oligosaccharide, in an amount of 0.5 to 1.5%,
- sodium cocoyl glutamate in an amount of 4 to 6%,
- safflower oil in an amount of 1 to 3%,
- argan oil in an amount of 1 to 3%, and
- hemp oil in an amount of 1 to 3%;

said composition being in particular a cleanser.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- KR 101308959 B1

- GB 2498543 A

**Littérature non-brevet citée dans la description**

- Gentle Foaming Cleanser Combination or Oily Skin. *Banque de données GNPD,* 01 Janvier 2009
- Deep Clear Washing Powder. *Banque de données GNPD,* 04 Juin 2019

- Facial Washing Powder. *Banque de données GNPD,* 18 Mars 2019
- Amazonian Ritual Powder Shampoo. *Banque de données GNPD,* 02 Septembre 2016
- *CHEMICAL ABSTRACTS,* 4430-18-6